# EUROPEAN PATENT APPLICATION

(11) **EP 3 884 957 A1**
(43) Date of publication of application: **29.09.2021**
(21) Application number: 20166052.9
(22) Date of filing: 26.03.2020
(51) Int. Cl.: A61K 38/48, A61K 38/17, A61P 31/14, A61P 11/00

(54) **METHODS FOR TREATMENT OF VIRUS AND METHODS FOR SCREENING OF ANTI-VIRUS REAGENT USING ORGANOIDS**

(71) Applicant: The University of British Columbia, Vancouver, British Columbia V6T 1Z3 (CA); Apeiron Biologics AG, 1030 Wien (AT); Institute for Bioengineering of Catalonia, 08028 Barcelona (ES); Catalan Institution for Research and Advanced Studies, 08010 Barcelona (ES); Mirazimi, Ali, 11354 Stockholm (SE); IMBA-Institut für Molekulare Biotechnologie GmbH, 1030 Wien (AT)
(72) Inventor: The designation of the inventor has not yet been filed
(74) Representative: Lahrtz, Fritz

(57) **Abstract**

Provided herein are methods for treating or preventing infection of a subject by a virus that infects the subject through angiotensin converting enzyme 2 and methods for screening of anti-virus reagent and vaccine candidates using organoids.

## Description

### FIELD OF THE INVENTION

The present disclosure generally relates to a method for treating or preventing infection of a subject by a virus that infects the subject through angiotensin converting enzyme 2 (ACE2), and a method for screening of anti-virus reagent using organoids.

### BACKGROUND

Outbreaks of emerging infectious diseases continue to challenge human health. The reported incidence of emerging and re-emerging zoonotic disease is increasing in many parts of the world. The Severe Acute Respiratory Syndrome Coronavirus (SARS-CoV) first emerged 17 years ago (Drosten et al., Identification of a novel coronavirus in patients with severe acute respiratory syndrome, N Engl J Med (2003) 348, 1967-1976). In December of 2019, a novel coronavirus (Severe Acute Respiratory Syndrome Coronavirus 2, "SARS-CoV-2") crossed species barriers to infect humans and was effectively transmitted from person to person, leading to a pneumonia outbreak. The SARS-CoV-2 causes coronavirus disease-19 (COVID-19) with influenza like symptoms ranging from mild disease to severe lung injury and multi-organ failure, eventually leading to death, especially in older patients with other co-morbidities.

SARS-CoV-2 is a positive-sense single-stranded RNA (+ssRNA) virus which belongs to the beta-coronavirus family and shares substantial genetic and functional similarity with other pathogenic human beta-coronaviruses, including Severe Acute Respiratory Syndrome Coronavirus ("SARS-CoV", also called SARS-CoV-1) and Middle East Respiratory Syndrome Coronavirus ("MERS-CoV"). Like other coronaviruses, SARS-CoV-2 has four structural proteins, known as the S (spike), E (envelope), M (membrane), and N (nucleocapsid) proteins. The spike protein is the protein responsible for allowing the SARS-CoV-2 virus to attach to the membrane of a host cell, the receptor binding domain ("RBD") of the spike protein of SARS-CoV-2 recognizes and attaches to the angiotensin-converting enzyme 2 ("ACE2") receptor of host cells to use them as a mechanism of cell entry.

ACE2 is a key metalloprotease of the Renin Angiotensin System (RAS), primarily existing as a membrane anchored zinc metalloprotease, which acts in the blood circulation and induces local effects in affected organs, as well as systemic effects via the blood circulation. ACE2 is expressed in the vascular system as well as in most organs, but predominantly in the lungs, kidneys, liver, heart, intestine and testes. In a normal adult human lung, ACE2 is expressed primarily in alveolar epithelial type II cells, which can serve as a viral reservoir. These cells produce surfactant which reduces surface tension, thus preventing alveoli from collapsing, and hence are critical to the gas exchange function of the lung. The overexpression of human ACE2 was identified to enhance disease severity in mice infected with SARS-CoV, demonstrating that ACE2-dependent viral entry into cells is a critical step (Yang et al., 2007). It was also reported that injecting SARS-CoV spike into mice decreased ACE2 expression levels, thereby worsening lung injury (Imai et al., 2005; Kuba et al., 2005). Thus, ACE2 serves both as the entry receptor of SARS-CoV and to protect the lung from injury.

A few disruptive agents targeted to ACE2 disrupting the RBD and ACE2 interaction have been shown to inhibit SARS-CoV infection. However, given the important physiological roles of ACE2 in vivo, these agents may have undesired side effects. Disruptive agents targeted to the RBD, on the other hand, are therefore more favorable.

No SARS-CoV-2-specific treatments or vaccine are currently available. Hence, an urgent need exists in the art to treat and/or prevent infection of a subject by a virus (especially Coronavirus), and there is also a great need for the methods of identifying a reagent capable of treating or preventing infection by a virus.

### BRIEF SUMMARY OF THE INVENTION

Throughout the present disclosure, the articles "a," "an," and "the" are used herein to refer to one or to more than one (i.e., to at least one) of the grammatical object of the article.

In one aspect, the present disclosure provides a method for treating or preventing infection of a subject by a virus that infects the subject through ACE2, comprising administering an effective amount of a recombinant ACE2 (rACE2) to the subject.

In another aspect, the present disclosure provides use of ACE2 in the manufacture of a medicament for treating or preventing infection of a subject by a virus that infects the subject through ACE2.

In one yet another aspect, the present disclosure provides use of ACE2 for treating or preventing infection of a subject by a virus that infects the subject through ACE2.

In one aspect, the present disclosure provides an *in vitro* method for treating or preventing infection of cells or tissue by a virus that infects cells or tissue through ACE2, comprising administering an effective amount of a rACE2 to the cells or tissue. In some embodiments, the tissue is lung, blood vessel, intestine, kidney, liver, brain or heart. In some embodiments, the cells are lung cells, blood vessel cells, intestine cells, kidney cells, liver cells, brain cells or heart cells. In some embodiments, the cells are human cells or non-human mammalian cells.

In some embodiments, the present disclosure provides a method for treating infection of a subject by a virus that infects the subject through ACE2, comprising administering an effective amount of a rACE2 to the subject. In some embodiments, the present disclosure provides a method for preventing infection of a subject by a virus that infects the subject through ACE2, comprising administering an effective amount of a rACE2 to the subject.

In some embodiments, the virus is a member of the family of coronaviruses, influenza viruses, Ebola or Marburg virus, or pseudotyped viruses. In some embodiments, the virus is coronavirus. In some embodiments, the coronavirus is selected from the group consisting of SARS-CoV, SARS-CoV-2 and MERS-CoV. In another embodiments, the coronavirus is SARS-CoV-2.

In some embodiments, the ACE2 is recombinant ACE2. In some embodiments, the ACE2 is recombinant human ACE2. In another embodiment, the ACE2 is soluble ACE2. In yet another embodiment, the ACE2 is soluble recombinant human ACE2. In some embodiments, the ACE2 is glycosylated. In some embodiments, at least 85%, 88%, 90%, 92%, 95% or 99% of the possible N-glycosylation sites of the ACE2 are glycosylated. In some embodiments, the glycosyl groups of ACE2 are present in a total of at least 10, 11, 12, 13, 14, 15 or at least 16 sialic acid residues, preferably at least 14 sialic acid residues. In some embodiments, the rhACE2 comprising amino acid sequence of amino acid 18-740 of SEQ ID NO: 1. In some embodiments, the rhACE2 is at least 90%, 92%, 95%, 98%, or 99% identical to the amino acid sequence of SEQ ID NO: 2.

In some embodiments, the ACE2 is present in monomeric, dimeric form, or multimeric forms. In some embodiments, the ACE2 is present in dimeric form. In some embodiments, the ACE2 is present in homo-dimeric form. In another embodiments, the ACE2 is present in multimeric form. In some embodiments, a linker is used to dimerize or multimerize rhACE2. In some embodiments, the linker is Fc region. In some embodiments, the rhACE2 is GSK2586881.

In some embodiments, the subject is human.

In some embodiments, the method is for treating or preventing infection of a tissue in the subject by viruses. In some embodiments, the method is for treating or preventing infection of a tissue in the subject by coronavirus. In some embodiments, the method is for treating or preventing infection of a tissue in the subject by SARS-CoV-2. In some embodiments, the tissue is lung, blood vessel, intestine, kidney, liver, brain, or heart. In some embodiments, the infection of lung, blood vessel, intestine, liver, heart, brain, or kidney in the subject is prevented or treated by administering an effective amount of ACE2 to the subj ect.

In some embodiments, the virus infects a subject through ACE2 expressed on lung cells. In another embodiment, the virus infects a subject through ACE2 expressed on alveolar epithelial type II cells. In some embodiments, the virus infects a subject through ACE2 expressed on blood vessel cells. In another embodiments, the virus infects a subject through ACE2 expressed on capillary cells. In some embodiments, the virus infects a subject through ACE2 expressed on cardiac cells. In some embodiments, the virus infects a subject through ACE2 expressed on kidney cells. In some embodiments, the virus infects a subject through ACE2 expressed on intestine cells. In some embodiments, the virus infects a subject through ACE2 expressed on liver cells. In some embodiments, the virus infects a subject through ACE2 expressed on brain cells. In some embodiments, the virus infects a subject through ACE2 expressed on testis cells.

In some embodiment, the ACE2 is administered in a pharmaceutical composition to the subject. In some embodiment, the ACE2 is administered in a pharmaceutically acceptable formulation to the subject.

In some embodiment, the ACE2 is administered through intravenous, intradermal, intramuscular injection, oral administration, or inhalation.

In some embodiment, the rACE2 is administered at a dosage of 0.01-10 mg/kg. In some embodiment, the rACE2 is administered at a dosage of 0.01-1 mg/kg. In another embodiment, the rACE2 is administered at a dosage of 0.4 mg/kg. In some embodiments, the ACE2 is administered twice a day. In another embodiment, the ACE2 is administered once a day. In some embodiments, the ACE2 is administered consecutively for at least 3, 4, 5, 6, 7, 8, 9, 10, 11, 12, 13, 14 or 15 days. In another embodiment, the ACE2 is administered consecutively for no more than 3, 4, 5, 6, 7, 8, 9, 10, 11, 12, 13, 14 or 15 days.

In some embodiment, the method further comprises administering to the subject a second therapeutic agent. In some embodiment, the method further comprises administering to the subject a second therapeutic agent for treating infections or complications. In some embodiment, the second therapeutic agent is an anti-viral agent or anti-inflammatory. In some embodiment, the anti-viral agent is hydroxychloroquine. In some embodiments, the second therapeutic agent is an RNA dependent RNA polymerase inhibitor, a non-nucleoside reverse transcriptase inhibitor (NNRTI), nucleoside reverse transcriptase inhibitor (NRTI), purine nucleoside, antiviral interferon, adamantine antiviral compound, or any other suitable antiviral agent. In some embodiments, the second therapeutic agent is remdesivir, chloroquine, hydroxychloroquine, lopinavir, ritonavir, APN01, favilavir, mesalazine, toremifene, eplerenone, paroxetine, sirolimus, dactinomycin, irbesartan, emodin, mercaptopurine, melatonin, quinacrine, carvedilol, colchicine, camphor, equilin, oxymetholone, nafamosta, camostat, derivatives thereof, or any combination thereof.

In one aspect, the present disclosure also provides an *in vitro* method for identifying a reagent capable of treating or preventing infection by a virus, comprising step (a1) contacting a test reagent with organoids infected by the virus or (a2) contacting organoids with a test reagent and the virus; and step (b) evaluating the effect of the test reagent on the infection of the organoids by the virus.

In another aspect, the present disclosure also provides an *in vitro* method for predicting the effect of a reagent on treating or preventing infection by a virus, comprising step (a1) contacting the reagent with organoids infected by the virus or (a2) contacting organoids with the reagent and the virus; and step (b) evaluating the effect of the reagent on the infection of the organoids by the virus. In some embodiments, the method further comprises selecting the reagent as a potential reagent for treating or preventing infection by the virus, if the step (b) shows that the reagent can treat or prevent the infection of the organoids by the virus.

In yet another aspect, the present disclosure also provides an *in vitro* method for predicting the effect of a reagent on treating or preventing infection of a tissue by a virus, comprising step (a1) contacting the reagent with organoids of the tissue infected by the virus or (a2) contacting organoids with the reagent and the virus; and step (b) evaluating the effect of the reagent on the infection of the organoids of the tissue by the virus. In some embodiments, the method further comprises selecting the reagent as a potential reagent for treating or preventing infection of the tissue by the virus, if the step (b) shows that the reagent can treat or prevent the infection of the organoids of the tissue by the virus.

In one aspect, the present disclosure further provides an *in vitro* method for evaluating the infection of a tissue by a virus, comprising step (a) contacting organoids of the tissue with the virus; and step (b) evaluating the infection of the organoids of the tissue by the virus. In some embodiments, the method further comprises selecting the organoids as a target for screening test reagent for treating or preventing infection by the virus, if the step (b) shows that the virus infects the organoids. In some embodiments, the method further comprises screening reagent which is used for treating or preventing infection of the tissue by the virus, if the step (b) shows that the virus infects the organoids of the tissue.

In another aspect, the present disclosure also provides an *in vitro* method for screening a vaccine candidate for preventing infection of a subject by a virus, comprising step (a) contacting organoids with the vaccine candidate and the virus; and step (b) evaluating the competition of the vaccine candidate with the virus for binding or entry to the organoids. In some embodiments, the organoids are contacted with the vaccine candidate and the virus at the same time, or the organoids are contacted with the vaccine candidate first and then contacted with the virus. In some embodiments, the method further comprises selecting the vaccine candidate as a potential vaccine for preventing infection by the virus, if the step (b) shows that the vaccine candidate can prevent or reduce the binding or entry to the organoids by the virus.

In some embodiments, the organoids are selected from the group consisting of human vascular organoids, human kidney organoids, human intestinal organoids, human liver organoids, human lung organoids, or human cardiac organoids and human brain organoids.

In some embodiments, the organoids are human vascular organoids. In some embodiments, the human vascular organoids are derived from induced pluripotent stem cells, human embryonic stem cells, or committed progenitor cells. In some embodiments, the human vascular organoids are derived from induced pluripotent stem cells by using CHIR99021 to start mesoderm differentiation and then using VEGF-A to start vascular differentiation and embed the cell aggregates. In some embodiments, the human vascular organoids comprise CD31⁺ endothelial lining and PDGFR⁺ pericyte coverage.

In some embodiments, the organoids are human kidney organoids. In some embodiments, the human kidney organoids are derived from human ES cells, human iPS cells, or committed progenitor cells. In some embodiments, the human kidney organoids are derived from human ES cells by using FGF9, heparin, activin A and CHIR. In some embodiments, the human kidney organoids are Lotus Tetraglobus Lectin⁺, SCL3A1⁺, SCL27A2⁺ and SCL5A12⁺. In some embodiments, the human kidney organoids develop tubular structures. In some embodiments, the human kidney organoids express ACE2.

In some embodiments, the organoids are human intestinal organoids. In some embodiments, the human intestinal organoids are derived from primary human intestinal epithelial cells. In some embodiments, the human intestinal organoids express ACE2.

In some embodiments, the organoids are human lung organoids. In some embodiments, the human lung organoids are derived from primary human lung epithelial cells, human stem cells, human induced pluripotent stem cells or human committed progenitor cells. In some embodiments, the human lung organoids are derived from primary human lung epithelial cells. In some embodiments, the human lung organoids express ACE2.

In some embodiments, the organoids are the human brain organoids. In some embodiments, the human brain organoids are derived from primary human neural progenitor cells, human glial progenitor cells, human stem cells, human IPS cells or human committed progenitor cells. In some embodiments, the human brain organoids are derived from primary human neural progenitor cells and glial progenitor cells. In some embodiments, the human brain organoids express ACE2.

In some embodiments, the organoids are the human liver organoids. In some embodiments, the human liver organoids are derived from primary human hepatic epithelial cells, human stem cells, human IPS cells or human committed progenitor cells. In some embodiments, the human liver organoids are derived from primary human hepatic cells. In some embodiments, the human liver organoids express ACE2.

In some embodiments, the organoids are human cardiac organoids. In some embodiments, the human cardiac organoids are derived from primary human cardiac epithelial cells, human stem cells, human IPS cells or human committed progenitor cells. In some embodiments, the human cardiac organoids are derived from primary cardiac cells. In some embodiments, the human cardiac organoids express ACE2.

In some embodiments, the step (a1) is conducted 1, 2, 3, 4, 5, 6, 7, 8, 9, or 10 days post infection of the organoids by the virus. In some embodiments, the step (b) comprises detecting the existence of the virus in the organoids. In some embodiments, the step (b) comprises detecting the RNA of the virus or infectious viral particles in the organoids. In some embodiments, the step (b) comprises detecting the RNA of the virus or infectious viral particles in the organoids by qRT-PCR.

In some embodiments, the organoids express ACE2. In some embodiments, the virus infects the organoids through ACE2. In some embodiments, the virus is a member of the family of coronaviruses, influenza viruses, Ebola or Marburg virus, or pseudotyped viruses. In some embodiments, the coronavirus is selected from the group consisting of SARS-CoV, SARS-CoV-2 and MERS-CoV. In another embodiments, the coronavirus is SARS-CoV-2.

In some embodiments, the test reagent is small molecule, RNA, DNA, polypeptide, lipid or polysaccharide.

### BRIEF DESCRIPTION OF FIGURES

The following drawings form part of the present specification and are included to further demonstrate certain aspects of the present disclosure. The disclosure may be better understood by reference to one or more of these drawings in combination with the detailed description of specific embodiments presented herein.
**Figure 1A** shows an electron microscopy image of a viral particle of the isolated SARS-CoV-2. **Figure 1B** shows a phylogenetic tree mapping the isolated SARS-CoV-2 to clade A3. **Figure 1C** shows the sequence alignment of the Swedish SARS-CoV-2 with SARS-CoV-2 S and L types, which shows that the Swedish isolate belongs to the L type.
**Figures 2A-2B** are images of a human capillary organoids using light microscopy (magnifications x 10). **Figure 2C** is an image of a human capillary organoids immunostained by using anti-CD31 to detect endothelial cells and anti-PDGFRβ to detect pericytes (scale bars, 500 µm and 50 µm).

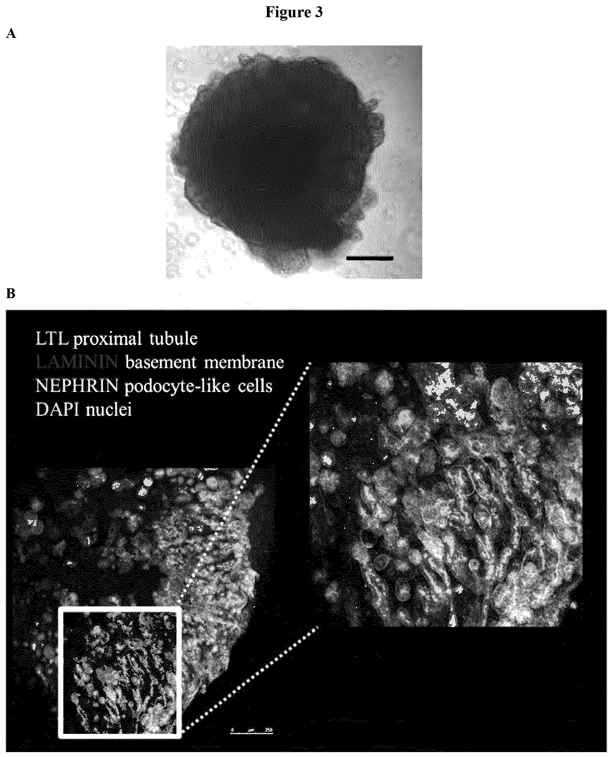

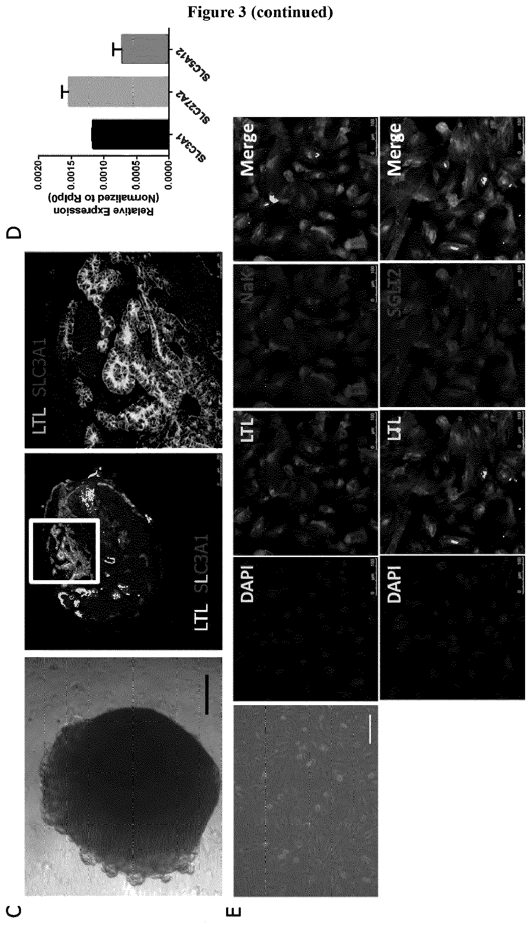

**Figure 3A** shows an image of a human kidney organoid at day 16 of differentiation visualized using light microscopy (scale bar 100 µm). **Figure 3B** is an image of a human kidney organoid at day 16 of differentiation visualized using confocal microscopy, showing tubular-like structures labelled with Lotus Tetraglobus Lectin (LTL, in green), podocyte-like cells showing positive staining for Nephrin (in turquoise), basement membrane marked by Laminin (in red) and nuclei labelled with DAPI (in green) (Scale bars 250 µm). A magnified view of the boxed region shows a detail of tubular structures (Scale bars 100 µm). **Figure 3C** are an image of a kidney organoid at day 16 of differentiation visualized using light microscopy (left, scale bar 100 µm) and confocal microscopy images of tubular-like structures labelled with LTL (in green) and the proximal tubular cell marker SCL3A1 (in red) (middle, scale bar 250 µm). A magnified view of the boxed region shows a detail of the tubular structures (right, scale bars 50 µm). **Figure 3D** is a bar chart showing the expression changes of SLC3A1, SLC5A12 and SLC27A2 of bulk samples at day 16 of organoid differentiation. **Figure 3E** shows LTL⁺ cells visualized using light microscopy (left, scale bar 100 µm) and LTL⁺ cells labelled visualized using confocal microscopy with LTL (in green), the proximal tubular cell markers NaK ATPase (NaK, in red), the solute carrier SGLT2 (in red) and nuclei (in blue) (scale bars 100 µm).
**Figure 4** shows an image of an intestinal organoid visualized by light microscopy (magnification x 10).
**Figure 5A** is a set of bar charts showing that the soluble rhACE2 inhibits SARS-CoV-2 infections of Vero-E6 cells, depending on the initial quantity of the virus in the inoculum and the dose of soluble rhACE2. **Figures 5B** is a set of bar charts showing that the equivalent recombinant mouse soluble ACE2 did not inhibit the infection.
**Figures 6A** is a set of bar charts showing the effect of the soluble rhACE2 treatment on progeny virus. **Figures 6B** is a set of bar charts showing the equivalent recombinant mouse soluble ACE2 did not inhibit the SARS-CoV-2 infections of Vero-E6 cells.
**Figure 7A** is a set of bar charts showing viral RNA from blood vessel organoids at day 3 and 6 post-infection (dpi) with SARS-CoV-2, demonstrating that the virus can infect the vascular organoids. **Figure 7B** is a bar chart showing that the infected human blood-vessels organoids produce progeny virus, depending of the initial level of infection. **Figure 7C** is a bar chart showing that the effect of soluble rhACE2 on SARS-CoV-2 infections of human blood vessel organoids.
**Figure 8A** is a bar chart showing that SARS-CoV-2 infects human tubular kidney organoids. **Figure 8B** is a bar chart showing that the infected kidney organoids produce progeny virus.
**Figure** 9 is a bar chart showing that SARS-CoV-2 infects human intestinal organoids.

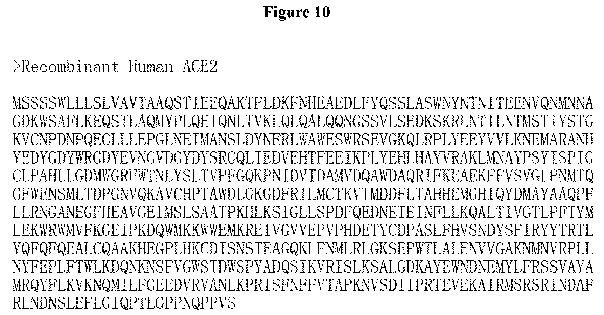

**Figure 10** shows the amino acid sequence of a human recombinant ACE2 (SEQ ID NO: 1).

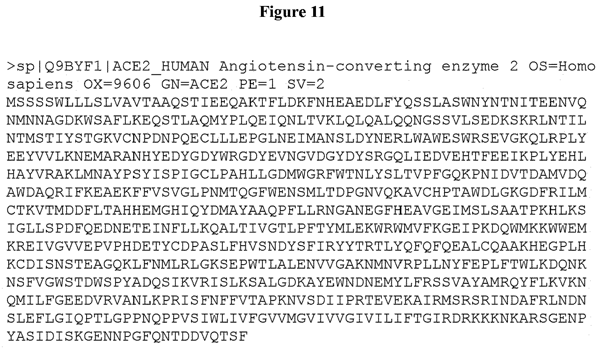

**Figure 11** shows the full-length amino acid sequence of human ACE2 (SEQ ID NO: 2). Amino acids 1-17 are the signal sequence, 18-740 form the extracellular domain, 741-761 form the transmembrane domain and 762-805 form the cytoplasmic domain.

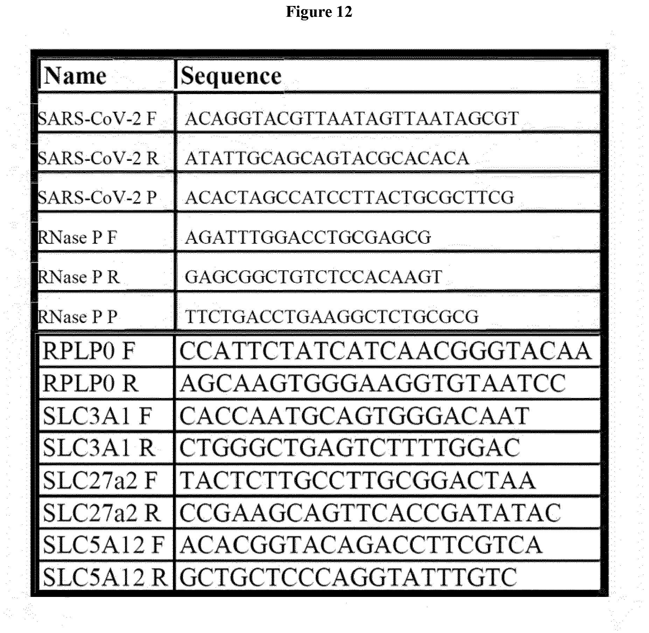

**Figure 12** shows a list of primers and probes used for RT-qPCR analysis (SEQ ID NOs: 3-16) in present studies.

### DETAILED DESCRIPTION OF THE INVENTION

The following description of the disclosure is merely intended to illustrate various embodiments of the disclosure. As such, the specific modifications discussed are not to be construed as limitations on the scope of the disclosure. It will be apparent to a person skilled in the art that various equivalents, changes, and modifications may be made without departing from the scope of the disclosure, and it is understood that such equivalent embodiments are to be included herein. All references cited herein, including publications, patents and patent applications are incorporated herein by reference in their entirety.

In one aspect, the present disclosure provides a method for treating or preventing infection of a subject by a virus that infects the subject through Angiotensin converting enzyme 2 (ACE2), comprising administering an effective amount of a recombinant Angiotensin converting enzyme 2 (rACE2) to the subject.

The terms "infection" used herein refers to the invasion of an organism's tissues or cells by viruses and related agents such as viroids and prions, their multiplication and reaction with host cells, as well as the resulted conditions and diseases.

The term "virus" as used herein refers to virus particles including the parental virus and its progeny virus, which may be infectious or noninfectious. The multiplication of the virus depend both on its destructive tendencies toward a specific host cell and on environmental conditions. In the vegetative cycle of viral infection, multiplication of progeny viruses can be rapid. This cycle of infection often results in the death of the cell and the release of many virus progeny.

The term "a virus that infects the subject through Angiotensin converting enzyme 2" as used herein refers to any virus, of which infection involves ACE2. In some embodiments, the virus uses ACE2 as receptor. In some embodiments, the virus uses ACE2 as receptor. In some embodiments, the virus uses ACE2 as receptor to enter into host cells. In some embodiments, the virus that infects the subject through ACE2 is a member of the family of coronaviruses, influenza viruses, Ebola or Marburg virus, or pseudotyped viruses. In some embodiments, the virus that infects the subject through ACE2 is Severe Acute Respiratory Syndrome Coronavirus 2 (SARS-CoV-2).

The term "ACE2" as used herein refers to a zinc metalloenzyme and carboxypeptidase located as an ectoenzyme on the surface of endothelial and other cells. While its primary substrate appears to be Ang II, it can hydrolyze a number of other physiological substrates. The human ACE2 (UniProt sequence data base entry: Q9BYF1) comprises a signal peptide (amino acids 1 to 17), an extracellular domain (amino acids 18 to 740), a helical transmembrane domain (amino acids 741 to 761) and a cytoplasmic domain (amino acids 762 to 805). In present invention, the ACE2 may be a full-length ACE2 polypeptide or a fragment thereof.

The term "recombinant" when used with reference to a polypeptide or a polynucleotide, refers to a polypeptide or polynucleotide that is produced by a recombinant method. A recombinant polypeptide includes any polypeptide expressed from a recombinant polynucleotide. A recombinant polynucleotide includes any polynucleotide which has been modified by the introduction of at least one exogenous (i.e., foreign, and typically heterologous) nucleotide or the alteration of at least one native nucleotide component of the polynucleotide, and need not include all of the coding sequence or the regulatory elements naturally associated with the coding sequence. A recombinant vector refers to a non-naturally occurring vector, including, e.g., a vector comprising a recombinant polynucleotide sequence.

In some embodiments, the rACE2 is a soluble rACE2. In one embodiment, the soluble rACE2 has no trans-membrane domains. In some embodiments, the soluble rACE2 comprises or consists of the amino acid sequence of the extracellular domain of ACE2 or any fragment thereof. In one embodiment, the soluble ACE2 comprises amino acids 374 to 378 of SEQ ID No: 2. In some embodiments, the soluble ACE2 comprises or consists of amino acids 18-740 of SEQ ID No: 2. In some embodiments, the soluble ACE2 consists of amino acids 18 to 615 of SEQ ID No: 2. In some embodiments, the soluble ACE2 is present in monomeric, dimeric, or multimeric forms.

The rACE2 used herein may optionally be glycosylated. The soluble domain of native human ACE2 has seven N-glycosylation sites. In one embodiment, 3, 4, 5, 6 or 7 of the possible N-glycosylation positions of the ACE2 polypeptides which correspond to Asn53, Asn90, Asn103, Asn322, Asn432, Asn546, Asn690 of SEQ ID NO: 2, are glycosylated and/or the ACE2 polypeptide has a sugar content of greater than 10% (percent by weight of the total ACE2 polypeptide) or 11%, 12%, 13%, 14%, 15%, 16%, 17%, 18%, 19%, in particular greater than 20%, 21%, 22%, 23%, 24%, or 25% by weight. In some embodiments, at least 85%, 88%, 90%, 92%, 95% or99% of the possible N-glycosylation sites of the rhACE2 polypeptide are glycosylated.

The term "sialic acid residues" as used herein, refers to residues of the N-acetylneuraminic acid type (Neu5Ac), especially N- glycosylation or O-glycosylation.

The rACE2 can be prepared by conventional methods well known in the art. The exemplary method for producing ACE2 polypeptides are described in WO2008/151347, the entire contents of which are incorporated herein by reference. The rACE2 could also be commercially available. In some embodiments, the rACE2 is GSK2586881.

The term "polypeptide" or "protein" means a string of at least two amino acids linked to one another by peptide bonds. Polypeptides and proteins may include moieties in addition to amino acids (e.g., may be glycosylated) and/or may be otherwise processed or modified. Those of ordinary skill in the art will appreciate that a "polypeptide" or "protein" can be a complete polypeptide chain as produced by a cell (with or without a signal sequence), or can be a functional portion thereof. Those of ordinary skill will further appreciate that a polypeptide or protein can sometimes include more than one polypeptide chain, for example a dimer. The dimer is preferably a homo-dimer, in particular, its monomer units have an identical sequence and glycosylation. In general, the monomer units of the dimer are non-covalently bound. The term also includes amino acid polymers in which one or more amino acids are chemical analogs of a corresponding naturally-occurring amino acid and polymers.

The term "nucleic acid" or "polynucleotide" as used herein refers to deoxyribonucleic acids (DNA) or ribonucleic acids (RNA) and polymers thereof in either single- or double-stranded form. Unless otherwise indicated, a particular polynucleotide sequence also implicitly encompasses conservatively modified variants thereof (e.g. degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (see Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

"Percent (%) sequence identity" with respect to amino acid sequence (or nucleic acid sequence) is defined as the percentage of amino acid (or nucleic acid) residues in a candidate sequence that are identical to the amino acid (or nucleic acid) residues in a reference sequence, after aligning the sequences and, if necessary, introducing gaps, to achieve the maximum number of identical amino acids (or nucleic acids). Conservative substitution of the amino acid residues may or may not be considered as identical residues. Alignment for purposes of determining percent amino acid (or nucleic acid) sequence identity can be achieved, for example, using publicly available tools such as BLASTN, BLASTp (available on the website of U.S. National Center for Biotechnology Information (NCBI), see also, Altschul S.F. et al., J. Mol. Biol., 215:403-410 (1990); Stephen F. et al., Nucleic Acids Res., 25:3389-3402 (1997)), ClustalW2 (available on the website of European Bioinformatics Institute, see also, Higgins D.G. et al., Methods in Enzymology, 266:383-402 (1996); Larkin M.A. et al., Bioinformatics (Oxford, England), 23(21): 2947-8 (2007)), and ALIGN or Megalign (DNASTAR) software. A person skilled in the art may use the default parameters provided by the tool, or may customize the parameters as appropriate for the alignment, such as for example, by selecting a suitable algorithm.

As used herein, the term "sample" used herein refers to a biological specimen that is obtained or derived from a subject of interest. The sample contains a cellular and/or other molecular entity that is to be characterized and/or identified, for example based on physical, biochemical, chemical and/or physiological characteristics.

The term "subject" as used herein refers to human and non-human animals infected or at a risk of being infected by a virus which infects the subject through ACE2. In some embodiments, the subject is human. Except when noted, the terms "patient" or "subject" are used herein interchangeably.

The term "pharmaceutically acceptable" indicates that the designated carrier, vehicle, diluent, excipient(s), and/or salt is generally chemically and/or physically compatible with the other ingredients comprising the formulation, and physiologically compatible with the recipient thereof.

The term "therapeutic effect" as used herein refers to a local or systemic effect in a subject, particularly humans, caused by a pharmacologically active substance. The term thus means any substance intended for use in the treatment or prevention of infection or disease, or in the enhancement of desirable physical or mental development and conditions in a subject.

The term "therapeutically effective amount" or "effective amount" used herein refers to an amount of an ACE2 that is effective for preventing, ameliorating and/or treating a condition resulting from infection with virus (preferably, SARS-CoV-2). "Amelioration" as used herein may refer to the reduction of visible or perceptible disease symptoms, viremia, or any other measurable manifestation of virus (preferably, SARS-CoV-2) infection. In one embodiment, a therapeutically effective amount of the ACE2 (i.e., an effective dosage) ranges from about 0.01-10 mg/kg body weight, preferably about 0.01-5 mg/kg body weight, more preferably about 0.1-1 mg/kg body weight, and even more preferably about 0.1-0.8 mg/kg, 0.2-0.7 mg/kg, 0.3-0.6 mg/kg or 0.4-0.5 mg/kg. The skilled artisan will appreciate that certain factors may influence the dosage required to effectively treat a subject, including but not limited to the severity of the infection, disease or disorder, previous treatments, the general health and/or age of the subject, and other diseases present.

Moreover, treatment of a subject with a therapeutically effective amount of ACE2 can include a single treatment or, preferably, can include a series of treatments. In some embodiments, a subject is treated with ACE2 in the range of between about 0.01-10 mg/kg body weight, one time per day or twice a day for between about 1 to 30 days. In some embodiments, the ACE2 is administered consecutively for at least 3, 4, 5, 6, 7, 8, 9, 10, 11, 12, 13, 14 or 15 days. In some embodiments, the ACE2 is administered consecutively for no more than 3, 4, 5, 6, 7, 8, 9, 10, 11, 12, 13, 14 or 15 days. It will also be appreciated that the effective dosage of ACE2 or the regimen used for treatment may be changed over the course of a particular treatment. Changes in dosage and/or regimen may result from the results of diagnostic assays.

The term "inhibit", "inhibiting" or "inhibition" include the decrease, limitation, or blockage, of, for example a particular action, function, or interaction. In some embodiments, infection is "inhibited" if at least one symptom of the infection is alleviated, terminated, slowed, or prevented. As used herein, infection is also "inhibited" if replication of the virus is reduced, slowed, delayed, or prevented.

The term "interaction", when referring to an interaction between two molecules, refers to the physical contact (e.g., binding) of the molecules with one another. Generally, such an interaction results in an activity (which produces a biological effect) of one or both of said molecules. The interaction between a rhACE2 and the RBD of spike protein of coronavirus (preferably SARS-CoV-2) may inhibit the binding interaction between the RBD of spike protein of coronavirus and its binding partner, such as the ACE receptor expressed on cells in a subject.

The term "treating" or "treatment" of an infection, disease, disorder or condition as used herein includes preventing or alleviating an infection, disease, disorder or condition, slowing the onset or rate of development of an infection, disease, disorder or condition, reducing the risk of developing an infection, disease, disorder or condition, preventing or delaying the development of symptoms associated with an infection, disease, disorder or condition, reducing or ending symptoms associated with an infection, disease, disorder or condition, generating a complete or partial regression of a disease, disorder or condition, curing a disease, disorder or condition, or some combination thereof. Those in need of treatment include those already inflicted with a condition resulting from infection of virus (preferably, SARS-CoV-2) as well as those in which infection with virus (preferably, SARS-CoV-2) is to be prevented. Subjects partially or totally recovered from infection of virus (preferably, SARS-CoV-2) might also be in need of treatment. Prevention encompasses inhibiting or reducing the spread of virus (preferably, SARS-CoV-2) or inhibiting or reducing the onset, development or progression of one or more of the symptoms associated with infection with virus (preferably, SARS-CoV-2).

The terms "prevent," "preventing," "prevention," "prophylactic treatment," and the like refer to reducing the probability of developing an infection, disease, disorder, or condition in a subject, who does not have, but is at risk of or susceptible to developing an infection, disease, disorder, or condition.

The present disclosure provides a method for treating or preventing infection of a subject by a virus that infects the subject through ACE2, comprising administering an effective amount of a rACE2 in a pharmaceutical composition to the subject. In some embodiments, the pharmaceutical compositions comprising an effective amount of a rACE2 and one or more pharmaceutically acceptable carriers.

Pharmaceutical acceptable carriers for use in the pharmaceutical compositions disclosed herein may include, for example, pharmaceutically acceptable liquid, gel, or solid carriers, aqueous vehicles, non-aqueous vehicles, antimicrobial agents, isotonic agents, buffers, tonicity-adjusting agents, antioxidants, anesthetics, suspending/dispending agents, sequestering or chelating agents, diluents, adjuvants, excipients, or non-toxic auxiliary substances, other components known in the art, or various combinations thereof.

Suitable components may include, for example, fillers, binders, disintegrants, buffers, preservatives, lubricants, flavorings, thickeners, coloring agents, emulsifiers or stabilizers such as sugars and cyclodextrins. In some embodiments, the suitable buffers may include, for example, a phosphate buffer or a MES (2-(N-morpholino)ethane sulfonic acid) buffer.

To further illustrate, pharmaceutical acceptable carriers may include, for example, aqueous vehicles such as sodium chloride injection, Ringer's injection, isotonic dextrose injection, sterile water injection, or dextrose and lactated Ringer's injection, nonaqueous vehicles such as fixed oils of vegetable origin, cottonseed oil, corn oil, sesame oil, or peanut oil, antimicrobial agents at bacteriostatic or fungistatic concentrations, isotonic agents such as sodium chloride or dextrose, buffers such as phosphate or citrate buffers or MES (2-(N-morpholino)ethane sulfonic acid buffers, antioxidants such as sodium bisulfate, local anesthetics such as procaine hydrochloride, suspending and dispersing agents such as sodium carboxymethylcelluose, hydroxypropyl methylcellulose, or polyvinylpyrrolidone, emulsifying agents such as Polysorbate 80 (TWEEN-80), sequestering or chelating agents such as EDTA (ethylenediaminetetraacetic acid) or EGTA (ethylene glycol tetraacetic acid), ethyl alcohol, polyethylene glycol, propylene glycol, sodium hydroxide, hydrochloric acid, citric acid, or lactic acid. Antimicrobial agents utilized as carriers may be added to pharmaceutical compositions in multiple-dose containers that include phenols or cresols, mercurials, benzyl alcohol, chlorobutanol, methyl and propyl p-hydroxybenzoic acid esters, thimerosal, benzalkonium chloride and benzethonium chloride. Suitable excipients may include, for example, water, saline, dextrose, glycerol, or ethanol. Suitable non-toxic auxiliary substances may include, for example, wetting or emulsifying agents, pH buffering agents, stabilizers, solubility enhancers, or agents such as sodium acetate, sorbitan monolaurate, triethanolamine oleate, or cyclodextrin.

The pharmaceutical compositions can be a liquid solution, suspension, emulsion, pill, capsule, tablet, sustained release formulation, or powder. Oral formulations can include standard carriers such as pharmaceutical grades of mannitol, lactose, starch, magnesium stearate, polyvinyl pyrollidone, sodium saccharine, cellulose, magnesium carbonate, etc.

The form of pharmaceutical compositions depends on a number of criteria, including, but not limited to, route of administration, extent of disease, or dose to be administered. The pharmaceutical compositions can be formulated for intravenous, oral, nasal, rectal, percutaneous, or intramuscular administration. For example, dosage forms for intravenous administration, may be formulated as lyophilized powder or fluid formulation; dosage forms for nasal administration may conveniently be formulated as aerosols, solutions, drops, gels or dry powders. In accordance to the desired route of administration, the pharmaceutical compositions can be formulated in the form of tablets, capsule, pill, dragee, powder, granule, sachets, cachets, lozenges, suspensions, emulsions, solutions, syrups, aerosols (as a solid or in a liquid medium), spray, inhalant, or suppository.

In certain embodiments, the pharmaceutical compositions are formulated into an injectable composition. The injectable pharmaceutical compositions may be prepared in any conventional form, such as for example liquid solution, suspension, emulsion, or solid forms suitable for generating liquid solution, suspension, or emulsion. Preparations for injection may include sterile and/or non-pyretic solutions ready for injection, sterile dry soluble products, such as lyophilized powders, ready to be combined with a solvent just prior to use, including hypodermic tablets, sterile suspensions ready for injection, sterile dry insoluble products ready to be combined with a vehicle just prior to use, and sterile and/or non-pyretic emulsions. The solutions may be either aqueous or nonaqueous.

In certain embodiments, unit-dose parenteral preparations are packaged in an ampoule, a vial or a syringe with a needle. All preparations for parenteral administration should be sterile and not pyretic, as is known and practiced in the art.

Depending on the route of administration, the rACE2 can be coated with or disposed in a selected material to protect it from natural conditions which may detrimentally affect its ability to perform its intended function. It may be administered alone, or in conjunction with a pharmaceutically acceptable carrier. It also may be administered as a prodrug, which is converted to its active form *in vivo.*

The present disclosure provides methods for treating or preventing infection of a subject by a virus that infects the subject through ACE2, comprising administering to the subject an effective amount of a rACE2, or the pharmaceutical composition comprising an effective amount of a rACE2.

In certain embodiment, the infection being treated or prevented is or is caused by coronavirus. In certain embodiment, the infection being treated or prevented is caused by SARS-CoV-2. In certain embodiments, the subject is human.

The rACE2, or the pharmaceutical composition comprising an effective amount of a rACE2 may be administered by any route known in the art, such as for example parenteral (e.g. subcutaneous, intraperitoneal, intravenous, including intravenous infusion, intramuscular, or intradermal injection) or non-parenteral (e.g. oral, intranasal, intraocular, sublingual, rectal, or topical) routes.

In certain embodiments, the rACE2 may be administered alone or in combination an effective amount of a second therapeutic agent. In some embodiments, the second therapeutic agent is an anti-viral agent. In one embodiment, the anti-viral agent is hydroxychloroquine. In some embodiments, the second therapeutic agent is an RNA dependent RNA polymerase inhibitor, a non-nucleoside reverse transcriptase inhibitor (NNRTI), nucleoside reverse transcriptase inhibitor (NRTI), purine nucleoside, antiviral interferon, adamantine antiviral compound, or any other suitable antiviral agent. In certain embodiments, the second therapeutic agent is selected from the group consisting of remdesivir, chloroquine, hydroxychloroquine, lopinavir, ritonavir, APN01, favilavir, mesalazine, toremifene, eplerenone, paroxetine, sirolimus, dactinomycin, irbesartan, emodin, mercaptopurine, melatonin, quinacrine, carvedilol, colchicine, camphor, equilin, oxymetholone, nafamosta, camostat, derivatives thereof, or any combination thereof.

In certain of these embodiments, rACE2 may be administered simultaneously with the one or more additional therapeutic agents, and in certain of these embodiments the rACE2 and the additional therapeutic agent(s) may be administered as part of the same pharmaceutical composition. However, a rACE2 administered "in combination" with another therapeutic agent does not have to be administered simultaneously with or in the same composition as the agent. A rACE2administered prior to or after another agent is considered to be administered "in combination" with that agent as the phrase is used herein, even if the rACE2 and the second agent are administered via different routes. Where possible, additional therapeutic agents administered in combination with the rACE2 are administered according to the schedule listed in the product information sheet of the additional therapeutic agent, or according to the Physicians' Desk Reference 2003 (Physicians' Desk Reference, 57th Ed; Medical Economics Company; ISBN: 1563634457; 57th edition (November 2002)) or protocols well known in the art.

In certain of these embodiments, a rACE2 may be administrated to a subject at a dosage of about 0.01-10 mg/kg body weight, preferably about 0.01-5 mg/kg body weight, more preferably about 0.1-1 mg/kg body weight, and even more preferably about 0.1-0.8 mg/kg, 0.2-0.7 mg/kg, 0.3-0.6 mg/kg or 0.4-0.5 mg/kg. In certain of these embodiments, a rACE2 may be administrated to a subject consecutively for at least 3, 4, 5, 6, 7, 8, 9, 10, 11, 12, 13, 14 or 15 days. In another embodiments, a rACE2 may be administrated to a subject consecutively for no more than 3, 4, 5, 6, 7, 8, 9, 10, 11, 12, 13, 14 or 15 days. In certain of these embodiments, a rACE2 may be administrated to a subject twice a day.

Provides herein are in vitro methods for identifying a reagent capable of treating or preventing infection by a virus, comprising step (a1) contacting a test reagent with organoids infected by the virus or (a2) contacting organoids with a test reagent and the virus; and step (b) evaluating the effect of the test reagent on the infection of the organoids by the virus.

In certain embodiments, the organoids are human capillary organoids, human kidney organoids, human intestinal organoids, human liver organoids, human lung organoids, or human heart and brain organoids. The organoids can be derived from one or a few cells from a tissue, induced pluripotent stem cells, human embryonic stem cells, or committed progenitor cells depending on the type of desired organoids, by using suitable reagents in different stages and controlling the conditions during the process. For example, in order to produce human blood vessel organoids closely resembling human capillaries with a lumen, CD31⁺ endothelial lining, PDGFR+ pericyte coverage, as well as formation of a basal membrane, it is important to use CHI99021 with BMP-4 to induce mesoderm and VEGF-A to start vascular differentiation and embed the cell aggregates (see Wimmer et al., Generation of blood vessel organoids from human pluripotent stem cells, Nat Protoc 14, (2019) 3082-3100, and Wimmer et al., Human blood vessel organoids as a model of diabetic vasculopathy, Nature 565, (2019) 505-510).

In some embodiments, for step (a), the test reagent can be added prior to, or after the infection of organoids by a virus. The test reagent and the virus can also be contacted with the organoids simultaneously. In certain embodiments, the organoids were infected with viruses for 1, 2, 3, 4, 5, 6, 7, 8, 9, or 10 days before contacting the test reagent.

In certain embodiments, the infectious virus is coronavirus such as SARS-CoV, SARS-CoV-2 and MERS-CoV. In certain embodiments, the virus infects the organoids through ACE2 receptor expressed on the organoids.

In certain embodiments, the reagent to be tested is a small molecule compound, RNA, DNA or a polypeptide. The reagent to be tested can also be a composition containing the small molecule compound, RNA, DNA or a polypeptide. The small molecule compound used herein refers to a compound having a molecular weight of less than or equal to about 2 kDa. In some embodiments, the small molecule compound has the molecular weight of less than or equal to 1kDa, 800Da, 700Da, 600Da or 500Da.

In certain embodiments, the effect of the test reagent on the infection of the organoids can be evaluated by detecting the presence or level of the virus. The presence or level of a virus in a sample can be determined based on the detection of the presence or level of the complex of the virus. Any suitable methods can be used for such detection, for example, qRT-PCR, flow cytometry (e.g., FACS™), plaque assay, or immunoassays such as immunoblotting, immunofluorescence (IF), immunohistochemistry (IHC), Enzyme-linked Immunosorbant Assay (ELISA), enzyme immunoassay (EIA), and radioimmunoassay (RIA). For a review of immunological and immunoassay procedures, see Basic and Clinical Immunology (Stites & Terr eds., 7th ed. 1991). Moreover, the immunoassays can be performed in any of several configurations, which are reviewed extensively in Enzyme Immunoassay (Maggio, ed., 1980); and Harlow & Lane, supra. For a review of the general immunoassays, see also Methods in Cell Biology: Antibodies in Cell Biology, volume 37 (Asai, ed. 1993); Basic and Clinical Immunology (Stites & Terr, eds., 7th ed. 1991). In a preferable embodiment, the presence or level of a virus is determined based on the RNA of a virus by qRT-PCR. The level of virus can be determined, for example, by normalizing to a control value or to a standard curve. The control value can be predetermined, or determined concurrently.

The present application also provides an in vitro method for predicting the effect of a reagent on treating or preventing infection by a virus, comprising step (a1) contacting the reagent with organoids infected by the virus or (a2) contacting organoids with the reagent with organoids and then contacting the organoids with the virus; and step (b) evaluating the effect of the reagent on the infection of the organoids by the virus. The reagent to be tested would be selected as a potential reagent for treating or preventing infection if the step (b) shows that the reagent can treat or prevent the infection of the organoids by the virus.

The present application also provides an in vitro method for predicting the effect of a reagent on treating or preventing infection of a tissue by a virus, comprising step (a1) contacting the reagent with organoids infected by the virus or (a2) contacting organoids with the reagent with organoids and then contacting the organoids with the virus; and step (b) evaluating the effect of the reagent on the infection of the organoids by the virus. The reagent to be tested would be selected as a potential reagent for treating or preventing infection of a tissue if the step (b) shows that the reagent can treat or prevent the infection of the organoids of a tissue by the virus.

An in vitro method for evaluating the infection of a tissue by a virus is provided herein, comprising step (a) contacting organoids of the tissue with the virus; and step (b) evaluating the infection of the organoids of the tissue by the virus. The organoids to be evaluated would be selected for screening reagents used for treating or preventing infection of the tissue by the virus if the step (b) shows that the virus infects the organoids. In certain embodiments, the evaluation is determined based on the RNA of virus by using qRT-PCR.

An in vitro method for screening a vaccine candidate for preventing infection of a subject by a virus is also provided herein, comprising step (a) contacting organoids with the vaccine candidate and the virus; and step (b) evaluating the competition of the vaccine candidate with the virus for binding or entry to the organoids. The organoids are contacted with the vaccine candidate and the virus at the same time, or the organoids are contacted with the vaccine candidate first and then contacted with the virus. The vaccine candidate would be selected as a potential vaccine for preventing infection by the virus if step (b) shows that the vaccine candidate can prevent or reduce the binding or entry to the organoids by the virus.

The following examples are provided to better illustrate the claimed invention and are not to be interpreted as limiting the scope of the invention. All specific compositions, materials, and methods described below, in whole or in part, fall within the scope of the present invention. These specific compositions, materials, and methods are not intended to limit the invention, but merely to illustrate specific embodiments falling within the scope of the invention. One skilled in the art may develop equivalent compositions, materials, and methods without the exercise of inventive capacity and without departing from the scope of the invention. It will be understood that many variations can be made in the procedures herein described while still remaining within the bounds of the present invention. It is the intention of the inventors that such variations are included within the scope of the invention.

### EXAMPLE 1. Materials and Methods

### a. Isolation and culture of SARS-CoV-2 particles

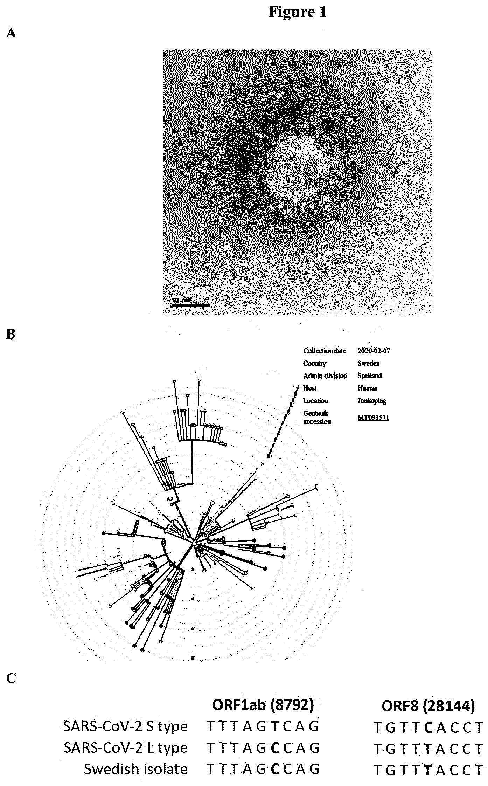

SARS-CoV-2 was isolated from a nasopharyngeal sample of a patient in Sweden with confirmed COVID-19 and cultured on Vero-E6 cells. The virus was titered using a plaque assay with fixation of cells 72 hours post infection as previously described (Becker et al., Synthetic recombinant bat SARS-like coronavirus is infectious in cultured cells and in mice, Proc Natl Acad Sci USA 105, (2008) 19944-19949). The isolated SARS-CoV-2 was sequenced by Next-Generation Sequencing (Genbank accession number MT093571). The cultured SARS-CoV-2 particles showed a prototypic coronal shape in electron microscopy (Figure 1A, viral stocks were inactivated using 35% Glutaraldehyde for electron microscopy) and belongs to the clad A3 by Phylogenetic analysis using GISAID (https://www.gisaid.org/) as shown in Figure 1B. For definition of the isolated Swedish SARS-CoV-2 as a part of S or L subtypes, as previously described (see Tang, X., Wu, C., Li, X., Song, Y., Yao, X., Wu, X., Duan, Y., Zhang, H., Wang, Y., Qian, Z., et al. (2020). On the origin and continuing evolution of SARS-CoV-2. National Science Review), nucleotide position 8782 (in orflab) was checked for a T (S type) or C (L type) nucleotide and position 28144 (in ORF8) was checked for a C (S type) or a T (L type) using Sequencher 5.4.6 software (Gene Codes Corporation). The results shows that this strain is a member of the L subtype (see Figure 1C).

### b. Recombinant human ACE2 and murine ACE2

The clinical-grade recombinant human ACE2 (amino acids 1-740) was produced by Polymun Scientific (contract manufacturer) from CHO cells according to Good Manufacturing Practice guidelines and formulated as a physiologic aqueous solution. The equivalent domain of murine ACE2 was similarly overexpressed in CHO cells under serum free conditions and purified by sequentially performing a capture step on DEAE-Sepharose, ammonium sulfate precipitation, purification via a HIC-Phenyl Sepharose column, followed by purification via a Superdex 200 gel filtration column. The purity of the murine protein was determined via HPLC, concentrations were determined with 280nm photometric measurements.

### c. Human capillary organoids

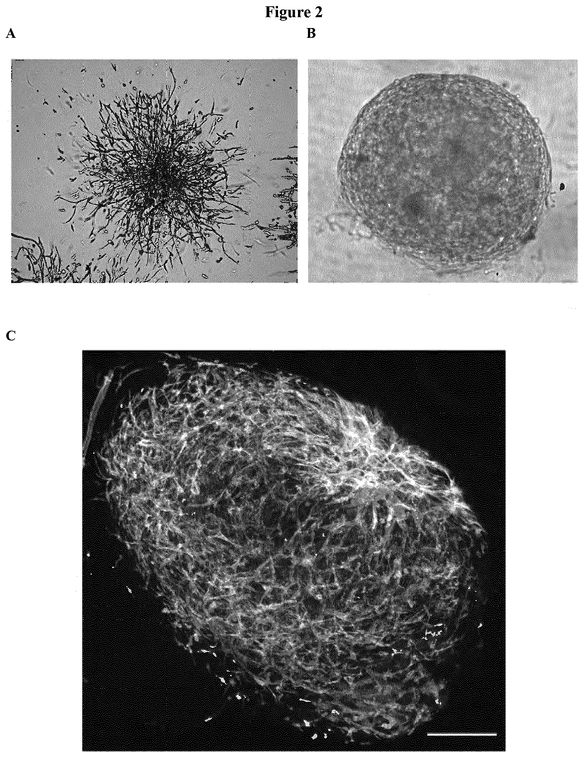

Human capillary organoids were engineered from induced pluripotent stem cells (iPSCs) and can be immunostained as previously described (see Wimmer et al., Generation of blood vessel organoids from human pluripotent stem cells, Nat Protoc 14, (2019) 3082-3100, the entire contents of which are incorporated herein by reference). The capillary organoids closely resemble human capillaries with a lumen, CD31⁺ endothelial lining, PDGFR⁺ pericyte coverage, as well as formation of a basal membrane (Wimmer et al., Human blood vessel organoids as a model of diabetic vasculopathy, Nature 565, (2019) 505-510). An exemplary image of vascular capillary organoids using light microscopy (magnifications x 10) is shown in Figure 2A-2B. The capillary organoids were immunostained by using anti-CD31 to detect endothelial cells and anti-PDGFRβ to detect pericytes (Figure 2C).

### d. Human kidney organoids

Human kidney organoids were engineered from human embryonic stem cells. The human embryonic stem cells were grown on vitronectin coated plates (1001-015, Life Technologies) and incubated with 0.5 mM EDTA (Merck) at 37 °C for 3 minutes for disaggregation. 100,000 cells/well were plated on a 24 multi-well plate coated with 5 µl/ml vitronectin and further incubated with supplemented Essential 8 Basal medium at 37 °C overnight. The day after (day 0), cells were treated for 3 subsequent days in Advanced RPMI 1640 basal medium (ThermoFisher Scientific) supplemented with 8 µM CHIR (Merck) and 1% Penicillin-Streptomycin and 1% of GlutaMAX TM (ThermoFisher Scientific). The medium was changed every day. From day 3 to 4, media were changed to Advanced RPMI supplemented with 200 ng/ml FGF9 (Peprotech), 1 µg/ml heparin (Merck) and 10 ng/ml activin A (Vitro). On day 4, cultures were rinsed twice with PBS, and resuspended in Advanced RPMI supplemented with 5 µM CHIR, 200 ng/ml FGF9 and 1 µg/ml Heparin. Cellular suspensions were seeded in V-shape 96 multi-well plate at a final concentration of 100,000 cells/well and centrifugated at 2000 rpm for 3 minutes. The resulting spheroids were incubated during 1 hour at 37 °C. Culture media was replaced by Advanced RPMI supplemented with 200 ng/ml FGF9 and 1 µg/ml Heparin for 7 additional days, the media was changed every second day. From day 11 to 16, developing organoids were incubated only in the presence of Advanced RPMI, the media was changed every second day. By histological analysis, the day 16 kidney differentiation organoids demonstrated prominent tubular-like structures as detected by using Lotus Tetraglobus Lectin (LTL) as a marker of proximal tubular epithelial cells (Figure 3B). The tubular-like cells also expressed the solute carrier SCL3A1 together with SCL27A2 and SCL5A12 (Figure 3C and Figure 3D).

For the isolation of LTL⁺ cells, kidney organoids were stained with fluorescein-conjugated LTL (FL-1321, Vector Laboratories). Then specimens were dissociated to single cells using Accumax (07921, Stem Cell Technologies) for 15 mins followed by 0.25% (wt/vol) trypsin (25300-054, Life Technologies) for 15 mins at 37 °C. FACSDiva software version 8.0.1 (BD Biosciences) was used in the FACS Aria Fusion instrument (BD Biosciences). By histological analysis, LTL positive (LTL⁺) cell fractions from day 16 kidney organoids was observed to express markers of proximal tubular identity (Figure 3E). The human kidney organoids could be regarded as a surrogate of human proximal tubule cell culture model.

### e. Exemplary histological analysis

Kidneys organoid/LTL⁺ cells were washed with PBS. Next samples were fixed with 4% paraformaldehyde (153799, Aname) for 20 mins at room temperature. Specimens were washed twice with PBS and further blocked using Tris-buffered saline (TBS) with 6% donkey serum (S30, Millipore) and 1% Triton X-100 (T8787, Sigma) for 1 hour at room temperature. After three rinses with antibody dilution buffer, samples were treated for 4 hours at room temperature with fluorescent conjugated secondary antibodies (Alexa Fluor (A) Cy3- or A647-; 1:200). A previous blocking step with a streptavidin/biotin blocking kit (SP-2002, Vector Labs) was performed for biotinylated LTL (B-1325, Vector Labs) and Alexa Fluor 488-conjugated streptavidin (SA5488, VectorLabs) to detect LTL⁺ cells. Antibodies to NEPHRIN (R&D SYSTEMS 4269; 1:100) and LAMININ (Sigma L9393; 1:50), SGLT2 (Abcam AB37296; 1:100), NaKATPase (Abcam; AB209299; 1:200) and SLC3A1 (Sigma HPA038360; 1:50) were used overnight at 4 °C diluted in antibody dilution buffer consisting of TBS with 6% donkey serum and 0.5% Triton X-100. Nuclei were detected using 4,6-diamidino-2-phenylindole (DAPI; 1:5000, D1306, Life Technologies) for 30mins. For mounting, samples were immersed in Fluoromount-G (0100-01, Southern Biotech). Sample confocal images were acquired with an SP5 Leica microscope and LTL⁺ were analysed using Image J.

### f. Human intestinal organoids

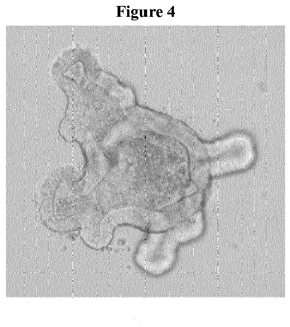

Human intestinal organoids were established from primary human intestinal epithelial cells (InEpC, Lonza). To establish intestinal organoids, 50,000 InEpC cells were suspended in a 50 µl Matrigel® dome and cultured with IntestiCult™ Organoid Growth Medium (STEMCELL Technologies). Media was supplemented with 10 µM Y-27632 (STEMCELL Technologies) for the first 3 days to improve organoid growth from a single cell suspension. Organoid cultures were clump passaged every 7 to 10 days according to manufacturer protocols (STEMCELL Technologies). An exemplary image of an intestinal organoid visualized by light microscopy (magnification x 10) is shown in Figure 4.

### g. Vero-E6 cells culture

Vero-E6 cells (ATCC) were grown in Dulbecco's Modified Eagle's Medium (DMEM, ThermoFisher Scientific) supplemented with 1% Non-Essential Amino-Acid (ThermoFisher Scientific), 10 mM Hepes (ThermoFisher Scientific) and 10% FBS at 37 °C, 5% CO₂.

### i. qRT-PCR for SARS CoV-2 RNA and Tubular Markers in Kidney Organoids

Samples were extracted using Direct-zol RNA MiniPrep kit (Zymo Research). qRT-PCR was performed using E-gene SARS-CoV-2 primers/probe following guidelines by the World Health Organization (https://www.who.int/docs/default-source/coronaviruse/wuhan-virus-assay-v1991527e51223 41d99287a1b17c111902.pdf). RNase P was used as an endogenous gene control to normalize the levels of intracellular viral RNA. The qRT-PCR for tubular markers in kidney organoids was conducted similarly using the corresponding primers.

**Table 1. Primers/probe used for qRT-PCR**

| **Primers/probe used for SARS-CoV-2** | | |
|---|---|---|
| Forward primer (FP) | ACAGGTACGTTAATAGTTAATAGCGT | SEQ ID NO: 3 |
| Reverse primer (RP) | ATATTGCAGCAGTACGCACACA | SEQ ID NO: 4 |
| Probe | FAM-ACACTAGCCATCCTTACTGCGCTTCG-QSY | SEQ ID NO: 5 |

| **Primers/probe used for RNase P** | | |
|---|---|---|
| FP | AGATTTGGACCTGCGAGCG | SEQ ID NO: 6 |
| RP | GAGCGGCTGTCTCCACAAGT | SEQ ID NO: 7 |
| Probe | FAM-TTCTGACCTGAAGGCTCTGCGCG-MGB | SEQ ID NO: 8 |

| **Primers used for tubular markers in kidney organoids** | | |
|---|---|---|
| RPLP0 FP | CCATTCTATCATCAACGGGTACAA | SEQ ID NO: 9 |
| RPLP0 RP | AGCAAGTGGGAAGGTGTAATCC | SEQ ID NO: 10 |
| SLC3A1 FP | CACCAATGCAGTGGGACAAT | SEQ ID NO: 11 |
| SLC3A1 RP | CTGGGCTGAGTCTTTTGGAC | SEQ ID NO: 12 |
| SLC27A2 FP | TACTCTTGCCTTGCGGACTAA | SEQ ID NO: 13 |
| SLC27A2 RP | CCGAAGCAGTTCACCGATATAC | SEQ ID NO: 14 |
| SLC5A12 FP | ACACGGTACAGACCTTCGTCA | SEQ ID NO: 15 |
| SLC5A12 RP | GCTGCTCCCAGGTATTTGTC | SEQ ID NO: 16 |

### Statistics

All statistical analyses were conducted using GraphPad Prism 8 (GraphPad) and significance was determined by Students t-test.

### EXAMPLE 2. Recombinant Human ACE2 inhibits SARS-CoV-2 infections in vitro

### 1. The soluble rhACE2 blocks SARS-CoV-2 infections of cells

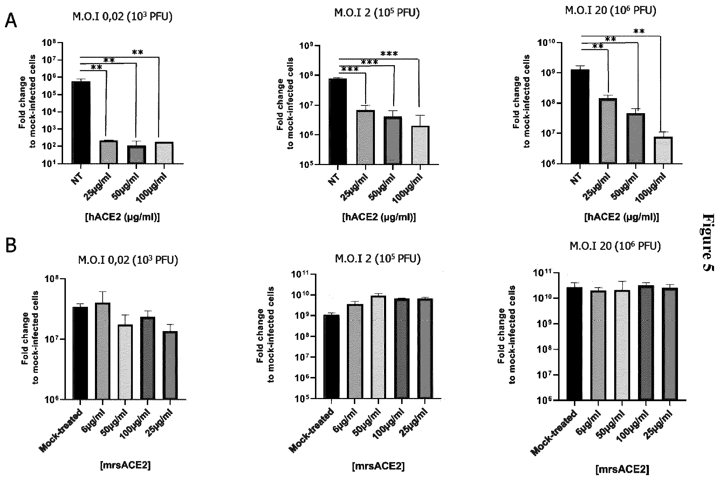

Vero E6 cells were seeded in 48-well plates (5 × 10⁴ cells per well) (Sarstedt) in DMEM containing 10% FBS for 24 hours. Different concentrations of soluble rhACE2 (25 µg/ml, 50 µg/ml and 100 µg/ml) were mixed with SARS-CoV-2 (v/v = 1:1) in a final volume of 100µl per well in DMEM (0% FBS) at 37°C. The SARS-CoV-2 was mixed at the amount of 10³ plaque forming units (PFUs, MOI 0.02), 10⁵ PFUs (MOI 2) and 10⁶ PFUs (MOI 20) respectively for each concentration. The mixture was then added to the culture medium of Vero-E6 cells. Cells were washed 3 times with PBS after 1 hour post-infection (h.p.i.) and incubated with fresh complete medium without soluble rhACE2. 15 hours post-infection, cells were washed 3 times with PBS and then lysed using Trizol™ (Thermofisher) before analysis by qRT-PCR for viral RNA detection. The SARS-CoV-2 RNA as a marker for replication was assayed by qRT-PCR. ** P<0.01; *** P<0.001. The results were shown in Figure 5A. The data demonstrates that soluble rhACE2 inhibits the attachment of the SARS-CoV-2 to the cells and this inhibition was dependent on the initial quantity of the virus in the inoculum and the dose of soluble rhACE2, establishing dose-dependency.

Vero E6 cells were seeded in 48-well plates (5 × 10⁴ cells per well) (Sarstedt) in DMEM containing 10% FBS for 24 hours. The equivalent soluble recombinant mouse ACE2 (soluble rmACE2, produced in the same way as soluble rhACE2) at a concentration of 6 µg/ml, 25 µg/ml, 50 µg/ml and 100 µg/ml respectively were mixed with SARS-CoV-2 (v/v = 1:1) in a final volume of 100 µl per well in DMEM (0% FBS) at 37 °C for 30 mins. The SARS-CoV-2 was mixed at the amount of 10³ PFUs (MOI 0.02), 10⁵ PFUs (MOI 2) and 10⁶ PFUs (MOI 20) respectively for each concentration. The mixture was then added to the culture medium of Vero-E6 cells. Cells were washed 3 times with PBS after 1 h.p.i and incubated with fresh complete medium without rmACE2. 15 hours post-infection, cells were washed 3 times with PBS and then lysed using Trizol™ (Thermofisher) before analysis by qRT-PCR for viral RNA detection. The viral RNA as a marker for replication was assayed by qRT-PCR. The results were shown in Figure 5B. The data demonstrates that soluble recombinant mouse ACE2 did not significantly affect SARS-CoV-2 infections of Vero-E6 cells, highlighting the specificity of soluble rhACE2 in blocking SARS-CoV-2 entry.

### 2. The soluble rhACE2 reduces SARS-CoV-2 infections

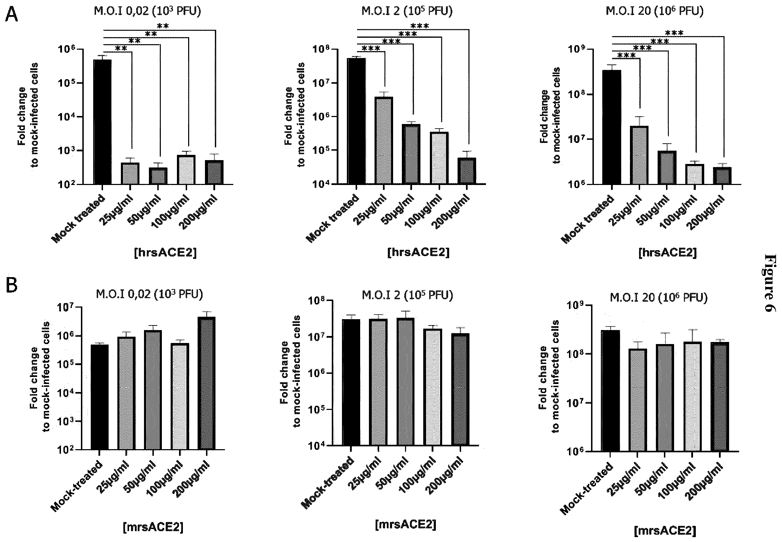

Vero E6 cells were seeded in 48-well plates (5 × 10⁴ cells per well) (Sarstedt) in DMEM containing 10% FBS for 24 hours. Different concentrations of soluble rhACE2 (25 µg/ml, 50 µg/ml, 100 µg/ml and 200 µg/ml) were mixed with SARS-CoV-2 (v/v = 1:1) in a final volume of 100 µl per well in DMEM (0% FBS) at 37 °C. The SARS-CoV-2 was mixed at the amount of 10³ PFUs (MOI 0.02), 10⁵ PFUs (MOI 2) and 10⁶ PFUs (MOI 20) respectively for each concentration. The mixture was then added to the culture medium of Vero-E6 cells. The inoculum was not removed. 15 hours post-infection, cells were washed 3 times with PBS and then lysed using Trizol™ (Thermofisher) before analysis by qRT-PCR for viral RNA detection. Viral RNA was assayed by qRT-PCR. The results were shown in Figure 6A. The significantly reduced virus infections was observed in the presence of soluble rhACE2 (Student t test: *P<0.05; ** P<0.01). It is probably because the soluble rhACE2 inhibits the progeny virus resulting in significantly reduced virus infections.

Vero E6 cells were seeded in 48-well plates (5 × 10⁴ cells per well) (Sarstedt) in DMEM containing 10% FBS for 24 hours. The equivalent soluble rmACE2 at a concentration of 25 µg/ml, 50 µg/ml, 100 µg/ml and 200 µg/ml respectively were mixed with SARS-CoV-2 (v/v = 1:1) in a final volume of 100µl per well in DMEM (0% FBS) at 37 °C for 30 mins. The SARS-CoV-2 was mixed at the amount of 10³ PFUs (MOI 0.02), 10⁵ PFUs (MOI 2) and 10⁶ PFUs (MOI 20) respectively for each concentration. The mixture was then added to the culture medium of Vero-E6 cells. The inoculum was not removed. 15 hours post-infection, cells were washed 3 times with PBS and then lysed using Trizol™ (Thermofisher) before analysis by qRT-PCR for viral RNA detection. Viral RNA was assayed by qRT-PCR. The results were shown in Figure 6B. The data demonstrates that soluble rmACE2 did not significantly affect SARS-CoV-2 infections of Vero-E6 cells, highlighting the specificity of soluble rhACE2 in reducing SARS-CoV-2 infections.

### 3. Cytotoxicity assay

To determine whether the recombinant human or mouse ACE2 are toxic to cells, 10⁴ Vero E6 cells per well were seeded in a 96-well plate. 24 hours post-seeding, 25 µl of different concentrations (from 25 to 200 µg/ml) of rACE2 were added in triplicate and incubated for 15 hours. 15 hours post-treatment, cytotoxicity was determined using the CellTiter-Glo® Luminescent cell viability assay (Promega) following the manufacturer's protocol. The addition of human or mouse rACE2 was not toxic to the Vero-E6 cells (data not shown).

### EXAMPLE 3. SARS-CoV-2 infects human blood vessel organoids and recombinant human ACE2 inhibits the infection

### SARS-CoV-2 infects human blood vessels organoids

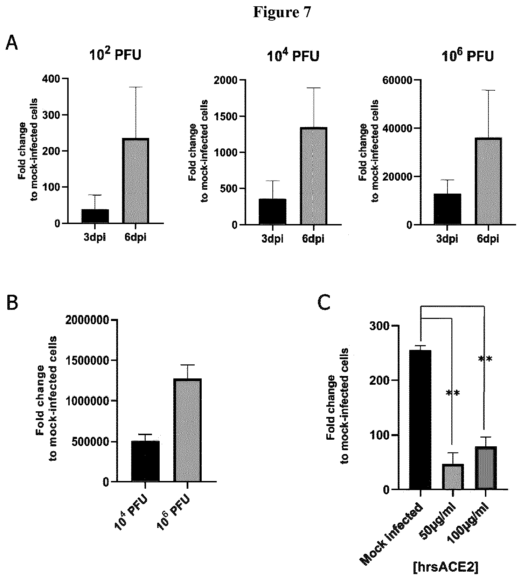

Groups of human blood vessel organoids were infected with 10², 10⁴, or 10⁶ SARS-CoV-2 infectious particles in StemPro complete media containing 15% FBS (Gibco cat. 10500064), 100 ng/ml of VEGF-A (Peprotech cat. no. 100-2) and 100 ng/ml of FGF-2 (Milteny Biotech cat. no. 130-093-841) in a volume of 50 µl per well of a 96-well ultra-low attachment plate for 1 hour. One hour post-infection, blood vessels organoids were washed 3 times with PBS and kept in 100 µl of corresponding medium (StemPro complete media containing 15% FBS, 100 ng/ml of VEGF-A and 100 ng/ml of FGF-2) for 3 to 6 days. On day 3 post-infection (3dpi), certain organoids were washed 3 times with PBS before being lysed with Trizol™ (Thermofisher). Samples were then analysed for the presence of SARS-CoV-2 RNA by qRT-PCR. On day 6 post-infection (6dpi), other organoids were washed 3 times with PBS before to lysis with Trizol™ (Thermofisher). Samples were then analysed for the presence of SARS-CoV-2 RNA by qRT-PCR. We could detect viral RNA in the blood vessel organoids with viral RNA increasing from day 3 to day 6 post infection (see Figure 7A), indicating active replication of SARS-CoV-2.

On day 6 post-infection, supernatants of the blood vessels organoids were recovered. 100 µl of each supernatant were used to infect Vero E6 in 48-well plate plates. Cells were recovered 48 hours post-infection and pooled (5 blood vessels organoids/condition), and the level of infection was determined by viral RNA detection using qRT-PCR. The supernatant of infected organoids collected at day 6 post-infection efficiently infected Vero E6 cells (Figure 7B) indicating that the infected blood-vessels organoids can produce infectious progeny virus, depending of the initial level of infection.

### Recombinant human ACE2 inhibits SARS-CoV-2 infection of human blood vessels organoids

Different concentrations of soluble rhACE2 (50 µg/ml and 100 µg/ml) were mixed with 10⁶ particles of SARS-CoV-2 for 30 mins at 37 °C in a final volume of 50 µl per well in STemPro 34 complete medium. The blood vessel organoids were then infected with the mixtures for 1 hour at 37 °C. Organoids were then washed 3 times with PBS and 100µl per well of new medium (StemPro complete media containing 15% FBS, 100 ng/ml of VEGF-A and 100 ng/ml of FGF-2) was added. Organoids were incubated for 3 days. To detect intracellular viral RNA, organoids were washed 3 times with PBS, pooled (5 organoids/condition) and lysed using Trizol™ (Thermofisher) before analysis. Then levels of SARS-CoV-2 RNA were assessed by qRT-PCR. The rhACE2 significantly decreased the level of SARS-CoV-2 infections in the vascular organoids (Student t test: ** P<0.01) as shown in Figure 7C. It can be seen that human blood vessel organoids can be readily infected with SARS-CoV-2 and this infection can be significantly inhibited by the rhACE2.

Those data evident that a primary site of SARS-CoV-2 infection appears to be the lung, and then SARS-CoV-2 infects blood vessels prior to local tissue (such as the kidney and intestine) infections.

### EXAMPLE 4. SARS-CoV-2 infects human kidney organoids

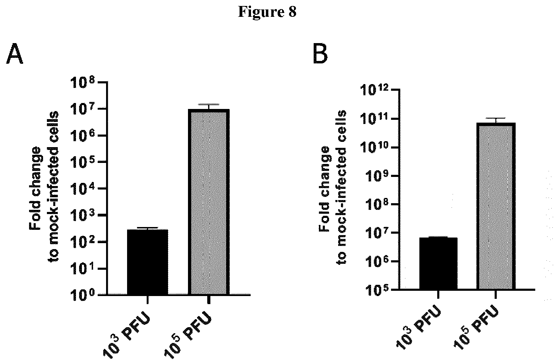

Groups of tubular kidney organoids were infected with 10³ or 10⁵ SARS-CoV-2 infectious particles in advanced RPMI medium (Thermofisher). One hour post-infection, tubular kidney organoids were washed 3 times with PBS and kept in 100µl advanced RPMI medium. Infections of the tubular kidney organoids were monitored 6 days after SARS-CoV-2 infection. The organoids were washed 3 times with PBS followed by lysed with Trizol™ (Thermofisher), and then were assayed for the presence of SARS-CoV-2 RNA by using qRT-PCR. The results were shown in Figure 8A.

On day 6 post-infection, supernatants of the tubular kidney organoids were recovered. 100 µl of each supernatant were used to infect Vero E6 in 48-well plate plates. Cells were recovered 48 hours post-infection and pooled (3 kidney organoids/condition), and the level of infection was determined by viral RNA detection using qRT-PCR. The results were shown in Figure 8B.

The SARS-CoV-2 was shown to directly infect the human kidney organoids and the infectious SARS-CoV-2 particles were recovered from the supernatants of the infected kidney organoids indicating that the infected kidney organoids can produce infectious progeny virus.

### EXAMPLE 5. SARS-CoV-2 infects human intestinal organoids

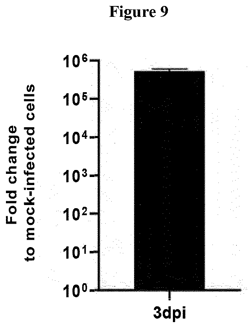

Intestinal organoids were removed from Matrigel matrix using EDTA 0.5 mM for 1 hour at 4 °C. And then were infected in suspension in IntestiCult™ OGM medium (STEMCELL Technologies) with 10⁶ SARS-CoV-2 infectious particles. 3 days post-infection, the intestinal organoids were collected by centrifugation, washed 3 times with PBS and lysed using Trizol™ (Thermofisher). The SARS-CoV-2 RNA levels inside the cells were assessed by using qRT-PCR. As shown in Figure 9, the SARS-Cov-2 RNA was actively replicated indicating that intestinal organoids can be readily infected by SARS-CoV-2.

To determine whether SARS-CoV2 can directly infect intestinal epithelium using gut organoids, these organoids were infected in suspension with 10⁶ SARS-CoV-2 infectious particles for 3 days. Viral RNA levels inside the cells were monitored by qRT-PCR. It shows intestinal organoids can be readily infected by SARS-CoV-2. These data provide direct evidence that besides blood vessels, engineered human kidneys and intestine can also be infected with SARS-CoV-2.

### EXAMPLE 6. SARS-CoV-2 infects human lung and heart organoids

Given the fact that the heart expresses high levels of ACE2 and heart alterations were the first phenotype observed in our *ace2* mutant mice (see Crackower, M.A., Sarao, R., Oudit, G.Y., Yagil, C., Kozieradzki, I., Scanga, S.E., Oliveira-dos-Santos, A.J., da Costa, J., Zhang, L., Pei, Y., et al. (2002). Angiotensin-converting enzyme 2 is an essential regulator of heart function. Nature 417, 822-828), lung and heart organoids may be involved in the multi-organ dysfunction in patients with COVID-19. Similar method of the present disclosure can be used to test infection of human heart organoids and lung organoids by SARS-CoV-2.

All of the methods and uses disclosed and claimed herein can be made and executed without undue experimentation in light of the present disclosure. While the methods and uses of this invention have been described in terms of preferred embodiments, it will be apparent to those of skill in the art that variations may be applied to the methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

### References

1. Drosten et al., Identification of a novel coronavirus in patients with severe acute respiratory syndrome, N Engl J Med 348, 1967-1976 (2003).
2. Yang, X.H. et al., Mice transgenic for human angiotensin-converting enzyme 2 provide a model for SARS coronavirus infection. Comp Med 57, 450-459 (2007).
3. Imai, Y. et al., Angiotensin-converting enzyme 2 protects from severe acute lung failure. Nature 436, 112-116 (2005).
4. Kuba, K. et al., A crucial role of angiotensin converting enzyme 2 (ACE2) in SARS coronavirus-induced lung injury. Nat Med 11, 875-879 (2005).
5. Wan, Y. et al., Receptor recognition by novel coronavirus from Wuhan: An analysis based on decade-long structural studies of SARS. J Virol (2020).
6. Batzer et al., Nucleic Acid Res. 19:5081 (1991).
7. Ohtsuka et al., J. Biol. Chem. 260: 2605-2608 (1985).
8. Rossolini et al., Mol. Cell. Probes 8: 91-98 (1994).
9. Altschul S.F. et al., J. Mol. Biol., 215:403-410 (1990).
10. Stephen F. et al., Nucleic Acids Res., 25:3389-3402 (1997).
11. Higgins D.G. et al., Methods in Enzymology, 266:383-402 (1996).
12. Larkin M.A. et al., Bioinformatics (Oxford, England), 23(21): 2947-8 (2007).
13. Becker et al., Synthetic recombinant bat SARS-like coronavirus is infectious in cultured cells and in mice, Proc Natl Acad Sci USA 105, 19944-19949 (2008).
14. Wimmer et al., Generation of blood vessel organoids from human pluripotent stem cells, Nat Protoc 14, 3082-3100 (2019).
15. Wimmer et al., Human blood vessel organoids as a model of diabetic vasculopathy, Nature 565, 505-510 (2019).
16. Chan, J.F. et al., Genomic characterization of the 2019 novel human-pathogenic coronavirus isolated from a patient with atypical pneumonia after visiting Wuhan. Emerg Microbes Infect 9, 221-236 (2020).
17. Clarke, N.E. et al., Angiotensin-converting enzyme 2: the first decade. Int J Hypertens, 307315 (2012).
18. Crackower, M.A. et al., Angiotensin-converting enzyme 2 is an essential regulator of heart function. Nature 417, 822-828 (2002).
19. Danilczyk, U. et al., J.MAngiotensin-converting enzyme II in the heart and the kidney. Circ Res 98, 463-471. (2006).
20. Ding, Y. et al., Organ distribution of severe acute respiratory syndrome (SARS) associated coronavirus (SARS-CoV) in SARS patients: implications for pathogenesis and virus transmission pathways. J Pathol 203, 622-630 (2004).
21. Garreta, E. et al., Fine tuning the extracellular environment accelerates the derivation of kidney organoids from human pluripotent stem cells. Nat Mater 18, 397-405 (2019).
22. Gu, J. et al., Multiple organ infection and the pathogenesis of SARS. J Exp Med 202, 415-424. (2005).
23. Guan, W.J. et al., Clinical Characteristics of Coronavirus Disease 2019 in China. NEngl J Med (2020*).*
24. Hamming, I. et al., Tissue distribution of ACE2 protein, the functional receptor for SARS coronavirus. A first step in understanding SARS pathogenesis. J Pathol 203, 631-637 (2004).
25. Haschke, M. et al., Pharmacokinetics and pharmacodynamics of recombinant human angiotensin-converting enzyme 2 in healthy human subjects. Clin Pharmacokinet 52, 783-792 (2013).
26. Hashimoto, T. et al., ACE2 links amino acid malnutrition to microbial ecology and intestinal inflammation. Nature 487, 477-481 (2012).
27. Hoffmann, M. et al., SARS-CoV-2 Cell Entry Depends on ACE2 and TMPRSS2 and Is Blocked by a Clinically Proven Protease Inhibitor. Cell (2020).
28. Huang, C. et al., Clinical features of patients infected with 2019 novel coronavirus in Wuhan, China. Lancet 395, 497-506 (2020).
29. Jeffers, S.A. et al., CD209L (L-SIGN) is a receptor for severe acute respiratory syndrome coronavirus. Proc Natl Acad Sci USA 101, 15748-15753 (2004).
30. Jiang, S. et al., An emerging coronavirus causing pneumonia outbreak in Wuhan, China: calling for developing therapeutic and prophylactic strategies. EmergMicrobes Infect 9, 275-277 (2020).
31. Khan, A. et al., A pilot clinical trial of recombinant human angiotensin-converting enzyme 2 in acute respiratory distress syndrome. Crit Care 21, 234 (2017).
32. Li, F. et al., Structure of SARS Coronavirus Spike Receptor-Binding Domain Complexed with Receptor. Science 309, 1864-1868 (2005).
33. Li, W. et al., Angiotensin-converting enzyme 2 is a functional receptor for the SARS coronavirus. Nature 426, 450-454 (2003).
34. Ling, Y. et al., Persistence and clearance of viral RNA in 2019 novel coronavirus disease rehabilitation patients. Chin Med J (Engl) (2020).
35. Lu, R. et al., Genomic characterisation and epidemiology of 2019 novel coronavirus: implications for virus origins and receptor binding. Lancet 395, 565-574 (2020).
36. Peng, L. et al., 2019 Novel Coronavirus can be detected in urine, blood, anal swabs and oropharyngeal swabs samples. medRxiv, 2020.2002.2021.20026179. (2020).
37. Tang, X. et al., On the origin and continuing evolution of SARS-CoV-2. National Science Review (2020).
38. Treml, B. et al., Recombinant angiotensin-converting enzyme 2 improves pulmonary blood flow and oxygenation in lipopolysaccharide-induced lung injury in piglets. Crit Care Med 38, 596-601 (2010).
39. Wrapp, D. et al., Cryo-EM structure of the 2019-nCoV spike in the prefusion conformation. Science (2020).
40. Wu, Z., and McGoogan, J.M., Characteristics of and Important Lessons From the Coronavirus Disease 2019 (COVID-19) Outbreak in China: Summary of a Report of 72314 Cases From the Chinese Center for Disease Control and Prevention. JAMA (2020).
41. Yeung, M.L. et al., MERS coronavirus induces apoptosis in kidney and lung by upregulating Smad7 and FGF2. Nat Microbiol 1, 16004 (2016).
42. Young, B.E. et al., Epidemiologic Features and Clinical Course of Patients Infected With SARS-CoV-2 in Singapore. JAMA (2020).
43. Zhang, H. et al., Angiotensin-converting enzyme 2 (ACE2) as a SARS-CoV-2 receptor: molecular mechanisms and potential therapeutic target. Intensive Care Med (2020).
44. Zhang Y. et al., Isolation of 2019-nCoV from a Stool Specimen of a Laboratory-Confirmed Case of the Coronavirus Disease 2019 (COVID-19). In China CDC Weekly (2020).
45. Zhao, Y. et al., Single-cell RNA expression profiling of ACE2, the putative receptor of Wuhan 2019-nCov. bioRxiv, 2020.2001.2026.919985 (2020).
46. Zhou, P. et al., A pneumonia outbreak associated with a new coronavirus of probable bat origin. Nature (2020).
47. Zhu, N. et al., A Novel Coronavirus from Patients with Pneumonia in China, 2019. N Engl J Med 382, 727-733 (2020).

## Claims

1. A method for treating or preventing infection of a subject by a virus that infects the subject through Angiotensin converting enzyme 2 (ACE2), comprising administering an effective amount of a recombinant Angiotensin converting enzyme 2 (rACE2) to the subject.

2. The method of claim 1, wherein the virus is a member of the family of coronaviruses, influenza viruses, Ebola or Marburg virus, or pseudotyped viruses.

3. The method of claim 1, wherein the virus is Severe Acute Respiratory Syndrome Coronavirus 2 (SARS-CoV-2).

4. The method of claim 1, wherein the rACE2 is a soluble rACE2.

5. The method of claim 1, wherein the rACE2 is a recombinant human ACE2 (rhACE2).

6. The method of claim 5, wherein the rhACE2 is present in monomeric, dimeric, or multimeric forms.

7. The method of claim 1, wherein a linker is used to dimerize or multimerize rhACE2.

8. The method of claim 7, wherein the linker is Fc region.

9. The method of claim 5, wherein the rhACE2 comprising amino acid sequence of amino acid 18-740 of SEQ ID NO: 1.

10. The method of claim 5, wherein at least 85%, 88%, 90%, 92%, 95% or 99% of the possible N-glycosylation sites of the rhACE2 are glycosylated.

11. The method of claim 5, wherein the rhACE2 is GSK2586881.

12. The method of claim 5, wherein rhACE2 is at least 90%, 92%, 95%, 98%, or 99% identical to the amino acid sequence of SEQ ID NO: 2.

13. The method of claim 1, wherein the method is for treating or preventing infection of a tissue in the subject by viruses.

14. The method of claim 1, wherein the method is for treating or preventing infection of a tissue in the subject by coronavirus.

15. The method of claim 1, wherein the method is for treating or preventing infection of a tissue in the subject by SARS-CoV-2.

16. The method of any one of claims 13-15, wherein the tissue is lung, blood vessel, intestine, kidney, liver, brain, or heart.

17. The method of claim 1, wherein the subject is human.

18. The method of claim 1, wherein the rACE2 is administered through intravenous, intradermal, intramuscular injection, oral administration or inhalation.

19. The method of claim 18, wherein the rACE2 is administered at a dosage of 0.01-10 mg/kg.

20. The method of claim 19, wherein the rACE2 is administered at a dosage of about 0.4 mg/kg.

21. The method of claim 1, wherein the rACE2 is administered twice a day.

22. The method of claim 1 or 21, wherein the rACE2 is administered consecutively for at least 3, 4, 5, 6, 7, 8, 9, 10, 11, 12, 13, 14 or 15 days.

23. The method of claim 1 or 21, wherein the rACE2 is administered consecutively for no more than 3, 4, 5, 6, 7, 8, 9, 10, 11, 12, 13, 14 or 15 days.

24. The method of claim 1, wherein the infection of lung, blood vessels, intestine, liver, heart, brain, or kidney in the subject is prevented or treated by administering an effective amount of rACE2 to the subject.

25. The method of claim 1, further comprising administering an effective amount of a second therapeutic agent.

26. The method of claim 25, wherein the second therapeutic agent is an anti-viral agent or anti-inflammatory, optionally, the anti-viral agent is hydroxychloroquine.

27. Use of rACE2 in the manufacture of a medicament for treating or preventing infection of a subject by a virus that infects the subject through ACE2.

28. Use of rACE2 for treating or preventing infection of a subject by a virus that infects the subject through ACE2.

29. An *in vitro* method for treating or preventing infection of cells or tissue by a virus that infects cells or tissue through ACE2, comprising administering an effective amount of rACE2 to the cells or tissue.

30. The method of claim 29, wherein the cells are lung cells, blood vessel cells, intestine cells, kidney cells, liver cells, brain cells or heart cells.

31. The method of claim 30, wherein the cells are human cells or non-human mammalian cells.

32. The method of claim 29, wherein the tissue is lung, blood vessel, intestine, kidney, liver, brain or heart.

33. An *in vitro* method for identifying a reagent capable of treating or preventing infection by a virus, comprising step (a1) contacting a test reagent with organoids infected by the virus or (a2) contacting organoids with a test reagent and the virus; and step (b) evaluating the effect of the test reagent on the infection of the organoids by the virus.

34. The method of claim 33, wherein the organoids are human vascular organoids, human kidney organoids, human intestinal organoids, human liver organoids, human lung organoids, human cardiac organoids, or human brain organoids.

35. The method of claim 34, wherein the human vascular organoids are derived from induced pluripotent stem cells, human embryonic stem cells, or committed progenitor cells.

36. The method of claim 34, wherein the human vascular organoids are derived from induced pluripotent stem cells by using CHIR99021 to start mesoderm differentiation and then using VEGF-A to start vascular differentiation and embed the cell aggregates.

37. The method of claim 34, wherein the human vascular organoids comprise CD31⁺ endothelial lining and PDGFR⁺ pericyte coverage.

38. The method of claim 34, wherein the human kidney organoids are derived from human embryonic stem cells, human induced pluripotent stem cells, or committed progenitor cells.

39. The method of claim 34, wherein the human kidney organoids are derived from human embryonic stem cells by using FGF9, heparin, activin A and CHIR.

40. The method of claim 34, wherein the human kidney organoids are Lotus Tetraglobus Lectin⁺, SCL3A1⁺, SCL27A2⁺ and SCL5A12⁺.

41. The method of claim 34, wherein the human kidney organoids develop tubular structures.

42. The method of claim 34, wherein the human kidney organoids express ACE2.

43. The method of claim 34, wherein the human intestinal organoids are derived from primary human intestinal epithelial cells, human stem cells, human induced pluripotent stem cells or human committed progenitor cells.

44. The method of claim 34, wherein the human intestinal organoids are derived from primary human intestinal epithelial cells.

45. The method of claim 34, wherein the human intestinal organoids express ACE2.

46. The method of claim 34, wherein the human lung organoids are derived from primary human lung epithelial cells, human stem cells, human induced pluripotent stem cells or human committed progenitor cells.

47. The method of claim 34, wherein the human lung organoids are derived from primary human lung epithelial cells.

48. The method of claim 34, wherein the human lung organoids express ACE2.

49. The method of claim 34, wherein the human brain organoids are derived from primary human neural progenitor cells, human glial progenitor cells, human stem cells, human IPS cells or human committed progenitor cells.

50. The method of claim 34, wherein the human brain organoids are derived from primary human neural progenitor cells and glial progenitor cells.

51. The method of claim 34, wherein the human brain organoids express ACE2.

52. The method of claim 34, wherein the human liver organoids are derived from primary human hepatic epithelial cells, human stem cells, human IPS cells or human committed progenitor cells.

53. The method of claim 34, wherein the human liver organoids are derived from primary human hepatic cells.

54. The method of claim 34, wherein the human liver organoids express ACE2.

55. The method of claim 34, wherein the human cardiac organoids are derived from primary human cardiac epithelial cells, human stem cells, human IPS cells or human committed progenitor cells.

56. The method of claim 34, wherein the human cardiac organoids are derived from primary cardiac cells.

57. The method of claim 34, wherein the human cardiac organoids express ACE2.

58. The method of claim 33, wherein the step (a1) is conducted 1, 2, 3, 4, 5, 6, 7, 8, 9, or 10 days post infection of the organoids by the virus.

59. The method of claim 33, wherein the step (b) comprises detecting the existence of the virus in the organoids.

60. The method of claim 33, wherein the step (b) comprises detecting the RNA of the virus or infectious viral particles in the organoids.

61. The method of claim 33, wherein the step (b) comprises detecting the RNA of the virus or infectious viral particles in the organoids by RT-qPCR.

62. The method of claim 33, wherein the virus infects the organoids through ACE2.

63. The method of claim 33, wherein the virus is a member of the family of coronaviruses, influenza viruses, Ebola or Marburg virus, or pseudotyped viruses.

64. The method of claim 33, wherein the virus is SARS-CoV-2.

65. The method of claim 33, wherein the test reagent is a small molecule compound, RNA, DNA, polypeptide, lipid or polysaccharide.

66. An *in vitro* method for predicting the effect of a reagent on treating or preventing infection by a virus, comprising step (a1) contacting the reagent with organoids infected by the virus or (a2) contacting organoids with the reagent and the virus; and step (b) evaluating the effect of the reagent on the infection of the organoids by the virus.

67. The method of claim 66, further comprising selecting the reagent as a potential reagent for treating or preventing infection by the virus, if step (b) shows that the reagent can treat or prevent the infection of the organoids by the virus.

68. An *in vitro* method for predicting the effect of a reagent on treating or preventing infection of a tissue by a virus, comprising step (a1) contacting the reagent with organoids of the tissue infected by the virus or (a2) contacting organoids with the reagent and the virus; and step (b) evaluating the effect of the reagent on the infection of the organoids of the tissue by the virus.

69. The method of claim 68, further comprising selecting the reagent as a potential reagent for treating or preventing infection of the tissue by the virus, if step (b) shows that the reagent can treat or prevent the infection of the organoids of the tissue by the virus.

70. An *in vitro* method for evaluating the infection of a tissue by a virus, comprising step (a) contacting organoids of the tissue with the virus; and step (b) evaluating the infection of the organoids of the tissue by the virus.

71. The method of claim 70, further comprising selecting the organoids as a target for screening test reagent for treating or preventing infection by the virus, if step (b) shows that the virus infects the organoids.

72. The method of claim 70, further comprising screening reagent which is used for treating or preventing infection of the tissue by the virus, if step (b) shows that the virus infects the organoids of the tissue.

73. An *in vitro* method for screening a vaccine candidate for preventing infection of a subject by a virus, comprising step (a) contacting organoids with the vaccine candidate and the virus; and step (b) evaluating the competition of the vaccine candidate with the virus for binding or entry to the organoids.

74. The method of claim 73, wherein the organoids are contacted with the vaccine candidate and the virus at the same time, or the organoids are contacted with the vaccine candidate first and then contacted with the virus.

75. The method of claim 73, further comprising selecting the vaccine candidate as a potential vaccine for preventing infection by the virus, if step (b) shows that the vaccine candidate can prevent or reduce the binding or entry to the organoids by the virus.


SEQUENCE LISTING

<110> University of British Columbia
      Karolinska Institute
      Apeiron Biologics AG
      Institute for Bioengineering of Catalonia
      Catalan Institution for Research and Advanced Studies

<120> METHODS FOR TREATMENT OF VIRUS AND METHODS FOR SCREENING OF
ANTI-VIRUS REAGENT USING ORGANOIDS

<130> 077499-8002WO01

<160> 16

<170> PatentIn version 3.5

<210> 1
<211> 740
<212> PRT
<213> Artificial Sequence

<220>
<223> recombinant human ACE2

<400> 1

Met Ser Ser Ser Ser Trp Leu Leu Leu Ser Leu Val Ala Val Thr Ala
1               5                   10                  15

Ala Gln Ser Thr Ile Glu Glu Gln Ala Lys Thr Phe Leu Asp Lys Phe
            20                  25                  30

Asn His Glu Ala Glu Asp Leu Phe Tyr Gln Ser Ser Leu Ala Ser Trp
        35                  40                  45

Asn Tyr Asn Thr Asn Ile Thr Glu Glu Asn Val Gln Asn Met Asn Asn
    50                  55                  60

Ala Gly Asp Lys Trp Ser Ala Phe Leu Lys Glu Gln Ser Thr Leu Ala
65                  70                  75                  80

Gln Met Tyr Pro Leu Gln Glu Ile Gln Asn Leu Thr Val Lys Leu Gln
                85                  90                  95

Leu Gln Ala Leu Gln Gln Asn Gly Ser Ser Val Leu Ser Glu Asp Lys
            100                 105                 110

Ser Lys Arg Leu Asn Thr Ile Leu Asn Thr Met Ser Thr Ile Tyr Ser
        115                 120                 125

Thr Gly Lys Val Cys Asn Pro Asp Asn Pro Gln Glu Cys Leu Leu Leu
    130                 135                 140

Glu Pro Gly Leu Asn Glu Ile Met Ala Asn Ser Leu Asp Tyr Asn Glu
145                 150                 155                 160

Arg Leu Trp Ala Trp Glu Ser Trp Arg Ser Glu Val Gly Lys Gln Leu
                165                 170                 175

Arg Pro Leu Tyr Glu Glu Tyr Val Val Leu Lys Asn Glu Met Ala Arg
            180                 185                 190

Ala Asn His Tyr Glu Asp Tyr Gly Asp Tyr Trp Arg Gly Asp Tyr Glu
        195                 200                 205

Val Asn Gly Val Asp Gly Tyr Asp Tyr Ser Arg Gly Gln Leu Ile Glu
    210                 215                 220

Asp Val Glu His Thr Phe Glu Glu Ile Lys Pro Leu Tyr Glu His Leu
225                 230                 235                 240

His Ala Tyr Val Arg Ala Lys Leu Met Asn Ala Tyr Pro Ser Tyr Ile
                245                 250                 255

Ser Pro Ile Gly Cys Leu Pro Ala His Leu Leu Gly Asp Met Trp Gly
            260                 265                 270

Arg Phe Trp Thr Asn Leu Tyr Ser Leu Thr Val Pro Phe Gly Gln Lys
        275                 280                 285

Pro Asn Ile Asp Val Thr Asp Ala Met Val Asp Gln Ala Trp Asp Ala
    290                 295                 300

Gln Arg Ile Phe Lys Glu Ala Glu Lys Phe Phe Val Ser Val Gly Leu
305                 310                 315                 320

Pro Asn Met Thr Gln Gly Phe Trp Glu Asn Ser Met Leu Thr Asp Pro
                325                 330                 335

Gly Asn Val Gln Lys Ala Val Cys His Pro Thr Ala Trp Asp Leu Gly
            340                 345                 350

Lys Gly Asp Phe Arg Ile Leu Met Cys Thr Lys Val Thr Met Asp Asp
        355                 360                 365

Phe Leu Thr Ala His His Glu Met Gly His Ile Gln Tyr Asp Met Ala
    370                 375                 380

Tyr Ala Ala Gln Pro Phe Leu Leu Arg Asn Gly Ala Asn Glu Gly Phe
385                 390                 395                 400

His Glu Ala Val Gly Glu Ile Met Ser Leu Ser Ala Ala Thr Pro Lys
                405                 410                 415

His Leu Lys Ser Ile Gly Leu Leu Ser Pro Asp Phe Gln Glu Asp Asn
            420                 425                 430

Glu Thr Glu Ile Asn Phe Leu Leu Lys Gln Ala Leu Thr Ile Val Gly
        435                 440                 445

Thr Leu Pro Phe Thr Tyr Met Leu Glu Lys Trp Arg Trp Met Val Phe
    450                 455                 460

Lys Gly Glu Ile Pro Lys Asp Gln Trp Met Lys Lys Trp Trp Glu Met
465                 470                 475                 480

Lys Arg Glu Ile Val Gly Val Val Glu Pro Val Pro His Asp Glu Thr
                485                 490                 495

Tyr Cys Asp Pro Ala Ser Leu Phe His Val Ser Asn Asp Tyr Ser Phe
            500                 505                 510

Ile Arg Tyr Tyr Thr Arg Thr Leu Tyr Gln Phe Gln Phe Gln Glu Ala
        515                 520                 525

Leu Cys Gln Ala Ala Lys His Glu Gly Pro Leu His Lys Cys Asp Ile
    530                 535                 540

Ser Asn Ser Thr Glu Ala Gly Gln Lys Leu Phe Asn Met Leu Arg Leu
545                 550                 555                 560

Gly Lys Ser Glu Pro Trp Thr Leu Ala Leu Glu Asn Val Val Gly Ala
                565                 570                 575

Lys Asn Met Asn Val Arg Pro Leu Leu Asn Tyr Phe Glu Pro Leu Phe
            580                 585                 590

Thr Trp Leu Lys Asp Gln Asn Lys Asn Ser Phe Val Gly Trp Ser Thr
        595                 600                 605

Asp Trp Ser Pro Tyr Ala Asp Gln Ser Ile Lys Val Arg Ile Ser Leu
    610                 615                 620

Lys Ser Ala Leu Gly Asp Lys Ala Tyr Glu Trp Asn Asp Asn Glu Met
625                 630                 635                 640

Tyr Leu Phe Arg Ser Ser Val Ala Tyr Ala Met Arg Gln Tyr Phe Leu
                645                 650                 655

Lys Val Lys Asn Gln Met Ile Leu Phe Gly Glu Glu Asp Val Arg Val
            660                 665                 670

Ala Asn Leu Lys Pro Arg Ile Ser Phe Asn Phe Phe Val Thr Ala Pro
        675                 680                 685

Lys Asn Val Ser Asp Ile Ile Pro Arg Thr Glu Val Glu Lys Ala Ile
    690                 695                 700

Arg Met Ser Arg Ser Arg Ile Asn Asp Ala Phe Arg Leu Asn Asp Asn
705                 710                 715                 720

Ser Leu Glu Phe Leu Gly Ile Gln Pro Thr Leu Gly Pro Pro Asn Gln
                725                 730                 735

Pro Pro Val Ser
            740

<210>  2
<211>  805
<212>  PRT
<213>  Homo sapiens

<400>  2

<210>  3
<211>  26
<212>  DNA
<213>  Artificial Sequence

<220>
<223>  Primer

<400>  3
acaggtacgt taatagttaa tagcgt                                          26

<210>  4
<211>  22
<212>  DNA

<213>  Artificial Sequence

<220>
<223>  Primer

<400>  4
atattgcagc agtacgcaca ca                                                22


<210>  5
<211>  26
<212>  DNA
<213>  Artificial Sequence

<220>
<223>  Probe

<400>  5
acactagcca tccttactgc gcttcg                                            26


<210>  6
<211>  19
<212>  DNA
<213>  Artificial Sequence

<220>
<223>  Primer

<400>  6
agatttggac ctgcgagcg                                                    19


<210>  7
<211>  20
<212>  DNA
<213>  Artificial Sequence

<220>
<223>  Primer

<400>  7
gagcggctgt ctccacaagt                                                   20


<210>  8
<211>  23
<212>  DNA
<213>  Artificial Sequence

<220>
<223>  Probe

<400>  8
ttctgacctg aaggctctgc gcg                                               23


<210>  9
<211>  24
<212>  DNA
<213>  Artificial Sequence

<220>

<223>  Primer

<400>  9
ccattctatc atcaacgggt acaa                                              24


<210>  10
<211>  22
<212>  DNA
<213>  Artificial Sequence

<220>
<223>  Primer

<400>  10
agcaagtggg aaggtgtaat cc                                                22


<210>  11
<211>  20
<212>  DNA
<213>  Artificial Sequence

<220>
<223>  Primer

<400>  11
caccaatgca gtgggacaat                                                   20


<210>  12
<211>  20
<212>  DNA
<213>  Artificial Sequence

<220>
<223>  Primer

<400>  12
ctgggctgag tcttttggac                                                   20


<210>  13
<211>  21
<212>  DNA
<213>  Artificial Sequence

<220>
<223>  Primer

<400>  13
tactcttgcc ttgcggacta a                                                 21


<210>  14
<211>  22
<212>  DNA
<213>  Artificial Sequence

<220>
<223>  Primer

<400>  14

ccgaagcagt tcaccgatat ac                                                22

<210>  15
<211>  21
<212>  DNA
<213>  Artificial Sequence

<220>
<223>  Primer

<400>  15
acacggtaca gaccttcgtc a                                                 21

<210>  16
<211>  20
<212>  DNA
<213>  Artificial Sequence

<220>
<223>  Primer

<400>  16
gctgctccca ggtatttgtc                                                   20